# EUROPEAN PATENT SPECIFICATION

(11) **EP 3 956 013 B1**
(45) Date of publication and mention of the grant of the patent: **05.06.2024**
(21) Application number: 20791177.7
(22) Date of filing: 14.04.2020
(51) Int. Cl.: A61N 1/08, A61N 1/36, A61N 1/05, A61N 1/375, A61N 1/37

(54) **MAGNET MANAGEMENT MRI COMPATIBILITY BY SHAPE**
MRT-KOMPATIBILITÄT MIT MAGNETMANAGEMENT NACH FORM
GESTION DE LA COMPATIBILITÉ DES AIMANTS D'IRM

(30) Priority: 15.04.2019 US 201962834348 P
(43) Date of publication of application: 23.02.2022
(73) Proprietor: Cochlear Limited, Macquarie University, New South Wales 2109 (AU)
(72) Inventor: RIDLER, Oliver John, Macquarie University, New South Wales 2109 (AU)
(74) Representative: Grünecker Patent- und Rechtsanwälte PartG mbB
(86) International application number: PCT/IB2020/053520
(87) International publication number: WO 2020/212849

(56) References cited:
- WO-A1-2013/028390
- WO-A1-2017/105510
- WO-A1-2018/200347
- US-A1- 2005 062 567
- US-A1- 2016 361 537
- US-A1- 2018 133 486
- US-A1- 2018 243 571
- US-B2- 8 255 058

## Description

### BACKGROUND

Hearing loss, which may be due to many different causes, is generally of two types: conductive and sensorineural. Sensorineural hearing loss is due to the absence or destruction of the hair cells in the cochlea that transduce sound signals into nerve impulses. Various hearing prostheses are commercially available to provide individuals suffering from sensorineural hearing loss with the ability to perceive sound. One example of a hearing prosthesis is a cochlear implant.

Conductive hearing loss occurs when the normal mechanical pathways that provide sound to hair cells in the cochlea are impeded, for example, by damage to the ossicular chain or the ear canal. Individuals suffering from conductive hearing loss may retain some form of residual hearing because the hair cells in the cochlea may remain undamaged.

Individuals suffering from hearing loss typically receive an acoustic hearing aid. Conventional hearing aids rely on principles of air conduction to transmit acoustic signals to the cochlea. In particular, a hearing aid typically uses an arrangement positioned in the recipient's ear canal or on the outer ear to amplify a sound received by the outer ear of the recipient. This amplified sound reaches the cochlea causing motion of the perilymph and stimulation of the auditory nerve. Cases of conductive hearing loss typically are treated by means of bone conduction hearing aids. In contrast to conventional hearing aids, these devices use a mechanical actuator that is coupled to the skull bone to apply the amplified sound.

In contrast to hearing aids, which rely primarily on the principles of air conduction, certain types of hearing prostheses, commonly referred to as cochlear implants, convert a received sound into electrical stimulation. The electrical stimulation is applied to the cochlea, which results in the perception of the received sound.

The international patent application WO 2018/200347 A1 relates to an MRI-safety and force optimized implant magnet system, and discloses all features in the preamble of claim 1.

### SUMMARY

The invention is defined by the appended claims.

In accordance with an exemplary embodiment, there is an implantable medical device, comprising a magnet apparatus and a support body supporting the magnet apparatus, wherein the magnet apparatus has a long axis and a short axis shorter than the long axis normal to the long axis, and at least one of the top surface or the bottom surface of the magnet apparatus establishes a curved outer periphery with respect to a cross-section lying on a plane on which the long axis lies and which is parallel to the short axis.

In an exemplary embodiment, there is an implantable medical device, comprising a non-spherical magnet apparatus, and a support body supporting the magnet apparatus, wherein
the device is configured to enable the magnet apparatus to rotate relative to the support body when exposed to an external magnetic field such that a magnetic field of the magnet apparatus aligns more with the external magnetic field relative to that which would otherwise be the case, and at least one of the magnet apparatus is a modified sphere shape or the magnet apparatus is configured to rotate relative to the support body about more than one axis.

In an exemplary embodiment, there is an implantable medical device, comprising a support body, and a magnet apparatus, wherein the support body includes a portion made of an elastomeric material that at least partially envelops the magnet apparatus and elastically deforms to enable the magnet apparatus to rotate about an axis parallel to a base of the device at least 45 degrees from a relaxed orientation when subjected to a magnetic field of at least 1 T that is oriented normal to a north-south magnetic axis of the magnet apparatus and normal to the axis that is parallel to the base.

In an exemplary embodiment, there is a method, comprising subjecting a subcutaneous medical device containing a magnet to a magnetic field of at least 1 T, thereby imparting a torque onto the magnet, the torque being about an axis that is parallel to surface of skin of the recipient and changing a thickness of the medical device in a direction normal to the axis by an increase of no less than 1 mm, thereby reducing the resulting torque on the overall medical device about the axis.

### BRIEF DESCRIPTION OF THE FIGURES

FIGs. 1A-4C present schematics of an exemplary implant in which embodiments of the teachings herein can be implemented;
FIGs. 5 and 6B present schematics for conceptual purposes;
FIGs. 7 and 8 present an exemplary embodiment of a magnet apparatus;
FIG. 9 presents an exemplary embodiment of an implant;
FIGs. 10-14 present operation of the implant when exposed to a pertinent magnetic field;
FIGs. 15-19 present schematics of features of some embodiments;
FIGs. 20-22 present exemplary magnet apparatuses;
FIG. 23 presents an exemplary embodiment of the embodiment of FIG. 1C but from a side view;
FIG. 24 is an exemplary flowchart for an exemplary non-claimed method;
FIGs. 25 and 26 present exemplary components that have structure between the magnet apparatus and the elastomeric material of the top of the implant; and
FIGs. 27-32 represent additional magnet apparatuses according to some embodiments.

### DETAILED DESCRIPTION

Exemplary embodiments will be described in terms of a cochlear implant. That said, it is noted that the teachings detailed herein and/or variations thereof can be utilized with other types of hearing prosthesis, such as by way of example, bone conduction devices, DACI / DACS / middle ear implants, etc. Still further, it is noted that the teachings detailed herein and/or variations thereof can be utilized with other types of prostheses, such as pacemakers, muscle stimulators, retinal implants, etc. In some instances, the teachings detailed herein and/or variations thereof are applicable to any type of implanted component (herein referred to as a medical device) having a magnet that is implantable in a recipient, such as, for example, pace makers, muscle stimulators, prosthetic limb actuators, components that transcutaneously transfer data and/or power, such as those that have utility with respect to aligning inductance coils for transmitting or receiving data to / from an implant, and/or charging an implant transcutaneous.

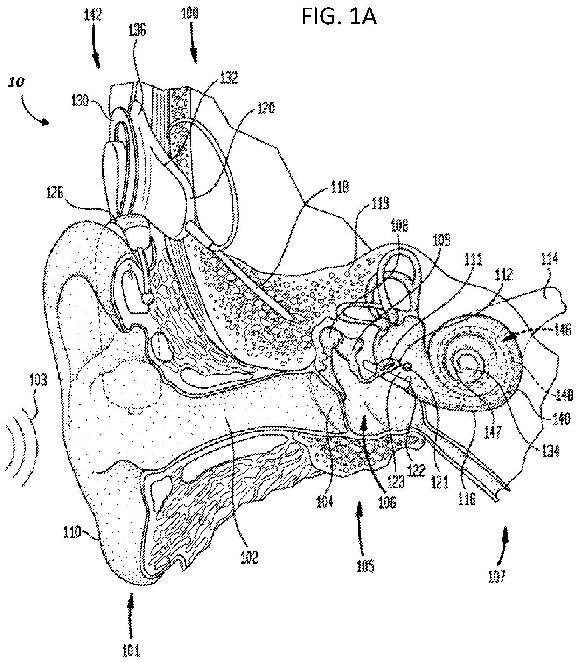

FIG. 1A is a perspective view of a cochlear implant, referred to as cochlear implant 100, implanted in a recipient, to which some embodiments detailed herein and/or variations thereof are applicable. The cochlear implant 100 is part of a system 10 that can include external components in some embodiments, as will be detailed below. It is noted that the teachings detailed herein are applicable, in at least some embodiments, to partially implantable and/or totally implantable cochlear implants (i.e., with regard to the latter, such as those having an implanted microphone). It is further noted that the teachings detailed herein are also applicable to other stimulating devices that utilize an electrical current beyond cochlear implants (e.g., auditory brain stimulators, pacemakers, etc.). Additionally, it is noted that the teachings detailed herein are also applicable to other types of hearing prostheses, such as by way of example only and not by way of limitation, bone conduction devices, direct acoustic cochlear stimulators, middle ear implants, etc. Indeed, it is noted that the teachings detailed herein are also applicable to so-called hybrid devices. In an exemplary embodiment, these hybrid devices apply both electrical stimulation and acoustic stimulation to the recipient. Any type of hearing prosthesis to which the teachings detailed herein and/or variations thereof that can have utility can be used in some embodiments of the teachings detailed herein.

In view of the above, it is to be understood that at least some embodiments detailed herein and/or variations thereof are directed towards a body-worn sensory supplement medical device (e.g., the hearing prosthesis of FIG. 1A, which supplements the hearing sense, even in instances where all natural hearing capabilities have been lost). It is noted that at least some exemplary embodiments of some sensory supplement medical devices are directed towards devices such as conventional hearing aids, which supplement the hearing sense in instances where some natural hearing capabilities have been retained, and visual prostheses (both those that are applicable to recipients having some natural vision capabilities remaining and to recipients having no natural vision capabilities remaining). Accordingly, the teachings detailed herein are applicable to any type of sensory supplement medical device to which the teachings detailed herein are enabled for use therein in a utilitarian manner. In this regard, the phrase sensory supplement medical device refers to any device that functions to provide sensation to a recipient irrespective of whether the applicable natural sense is only partially impaired or completely impaired.

The recipient has an outer ear 101, a middle ear 105 and an inner ear 107. Components of outer ear 101, middle ear 105, and inner ear 107 are described below, followed by a description of cochlear implant 100.

In a fully functional ear, outer ear 101 comprises an auricle 110 and an ear canal 102. An acoustic pressure or sound wave 103 is collected by auricle 110 and channeled into and through ear canal 102. Disposed across the distal end of ear channel 102 is a tympanic membrane 104 which vibrates in response to sound wave 103. This vibration is coupled to oval window or fenestra ovalis 112 through three bones of middle ear 105, collectively referred to as the ossicles 106 and comprising the malleus 108, the incus 109 and the stapes 111. Bones 108, 109, and 111 of middle ear 105 serve to filter and amplify sound wave 103, causing oval window 112 to articulate, or vibrate in response to vibration of tympanic membrane 104. This vibration sets up waves of fluid motion of the perilymph within cochlea 140. Such fluid motion, in turn, activates tiny hair cells (not shown) inside of cochlea 140. Activation of the hair cells causes appropriate nerve impulses to be generated and transferred through the spiral ganglion cells (not shown) and auditory nerve 114 to the brain (also not shown) where they are perceived as sound.

As shown, cochlear implant 100 comprises one or more components which are temporarily or permanently implanted in the recipient. Cochlear implant 100 is shown in FIG. 1A with an external device 142, that is part of system 10 (along with cochlear implant 100), which, as described below, is configured to provide power to the cochlear implant, and where the implanted cochlear implant includes a battery, that is recharged by the power provided from the external device 142.

In the illustrative arrangement of FIG. 1A, external device 142 can comprise a power source (not shown) disposed in a Behind-The-Ear (BTE) unit 126. External device 142 also includes components of a transcutaneous energy transfer link, referred to as an external energy transfer assembly. The transcutaneous energy transfer link is used to transfer power and/or data to cochlear implant 100. Various types of energy transfer, such as infrared (IR), electromagnetic, capacitive and inductive transfer, may be used to transfer the power and/or data from external device 142 to cochlear implant 100. In the illustrative embodiments of FIG. 1A, the external energy transfer assembly comprises an external coil 130 that forms part of an inductive radio frequency (RF) communication link. External coil 130 is typically a wire antenna coil comprised of multiple turns of electrically insulated single-strand or multi-strand platinum or gold wire. External device 142 also includes a magnet (not shown) positioned within the turns of wire of external coil 130. It should be appreciated that the external device shown in FIG. 1A is merely illustrative, and other external devices may be used with embodiments of the present invention.

Cochlear implant 100 comprises an internal energy transfer assembly 132 which can be positioned in a recess of the temporal bone adjacent auricle 110 of the recipient. As detailed below, internal energy transfer assembly 132 is a component of the transcutaneous energy transfer link and receives power and/or data from external device 142. In the illustrative embodiment, the energy transfer link comprises an inductive RF link, and internal energy transfer assembly 132 comprises a primary internal coil assembly 136. Internal coil assembly 136 typically includes a wire antenna coil comprised of multiple turns of electrically insulated single-strand or multi-strand platinum or gold wire, as will be described in greater detail below.

Cochlear implant 100 further comprises a main implantable component 120 and an elongate electrode assembly 118. Collectively, the coil assembly 136, the main implantable component 120, and the electrode assembly 118 correspond to the implantable component of the system 10.

In some embodiments, internal energy transfer assembly 132 and main implantable component 120 are hermetically sealed within a biocompatible housing. In some embodiments, main implantable component 120 includes an implantable microphone assembly (not shown) and a sound processing unit (not shown) to convert the sound signals received by the implantable microphone or via internal energy transfer assembly 132 to data signals. That said, in some alternative embodiments, the implantable microphone assembly can be located in a separate implantable component (e.g., that has its own housing assembly, etc.) that is in signal communication with the main implantable component 120 (e.g., via leads or the like between the separate implantable component and the main implantable component 120). In at least some embodiments, the teachings detailed herein and/or variations thereof can be utilized with any type of implantable microphone arrangement.

Main implantable component 120 further includes a stimulator unit (also not shown in FIG. 1A) which generates electrical stimulation signals based on the data signals. The electrical stimulation signals are delivered to the recipient via elongate electrode assembly 118.

Elongate electrode assembly 118 has a proximal end connected to main implantable component 120, and a distal end implanted in cochlea 140. Electrode assembly 118 extends from main implantable component 120 to cochlea 140 through mastoid bone 119. In some embodiments electrode assembly 118 may be implanted at least in basal region 116, and sometimes further. For example, electrode assembly 118 may extend towards apical end of cochlea 140, referred to as cochlea apex 134. In certain circumstances, electrode assembly 118 may be inserted into cochlea 140 via a cochleostomy 122. In other circumstances, a cochleostomy may be formed through round window 121, oval window 112, the promontory 123 or through an apical turn 147 of cochlea 140.

Electrode assembly 118 comprises a longitudinally aligned and distally extending array 146 of electrodes 148, disposed along a length thereof. As noted, a stimulator unit generates stimulation signals which are applied by electrodes 148 to cochlea 140, thereby stimulating auditory nerve 114.

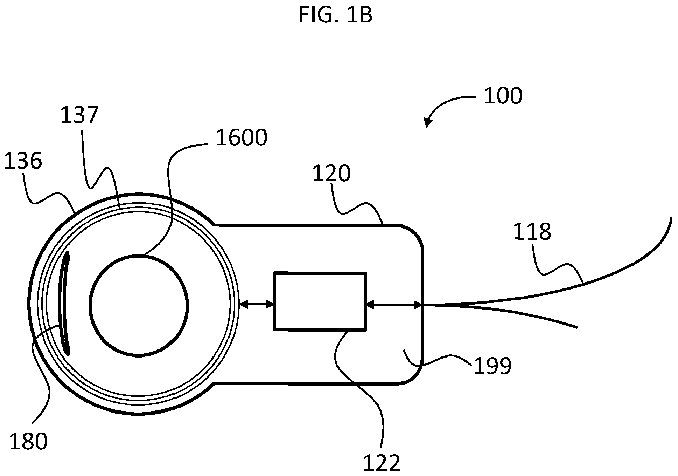

FIG. 1B depicts an exemplary high-level diagram of the implantable component 100 of the system 10, looking downward from outside the skull towards the skull. As can be seen, implantable component 100 includes a magnet apparatus 1600 that is surrounded by a coil 137 that is in two-way communication (although in other embodiments, the communication is one-way) with a stimulator unit 122, which in turn is in communication with the electrode assembly 118.

Still with reference to FIG. 1B, it is noted that the stimulator unit 122 and the magnet apparatus 1600 are located in a housing made of an elastomeric material 199, such as by way of example only and not by way of limitation, silicone. Hereinafter, the elastomeric material 199 of the housing will be often referred to as silicone. However, it is noted that any reference to silicone herein also corresponds to a reference to any other type of component that will enable the teachings detailed herein and/or variations thereof, such as, by way of example and not by way of limitation only, bio-compatible rubber, etc.

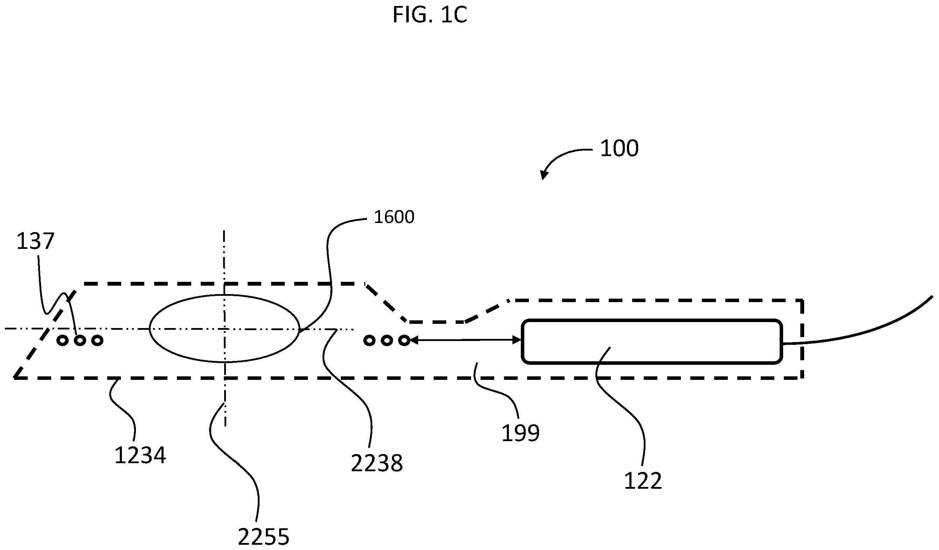

As can be seen in FIG. 1B, the housing made of elastomeric material 199 includes a slit 180 (not shown in FIG. 1C, as, in some embodiments, the slit is not utilized). In an exemplary embodiment, the slit 180 has utilitarian value in that it can enable insertion and/or removal of the magnet apparatus 1600 from the housing made of elastomeric material 199.

It is noted that magnet apparatus 1600 is presented in a conceptual manner. In this regard, it is noted that in at least some embodiments, the magnet apparatus 1600 is an assembly that includes a magnet surrounded by a biocompatible coating. Still further, in an exemplary embodiment, magnet apparatus 1600 is an assembly where the magnet is located within a container having interior dimensions generally corresponding to the exterior dimensions of the magnet of the magnet apparatus. This container can be hermetically sealed, thus isolating the magnet in the container from body fluids of the recipient that penetrate the housing (the same principle of operation occurs with respect to the aforementioned coated magnet). In an exemplary embodiment, this container moves with the magnet. Additional details of the container will be described below. In this regard, it is noted that sometimes the term magnet is used as shorthand for the phrase magnet apparatus, and visa-versa, and thus any disclosure herein with respect to a magnet also corresponds to a disclosure of a magnet apparatus according to the embodiments herein and/or variations thereof and/or any other configuration that can have utilitarian value according to the teachings detailed herein, and visa-versa.

With reference now to FIG. 1C, it is noted that the outlines of the housing made from elastomeric material 199 are presented in dashed line format for ease of discussion. In an exemplary embodiment, silicone or some other elastomeric material fills the interior within the dashed line, other than the other components of the implantable device (e.g., plates, magnet, stimulator, etc.). That said, in an alternative embodiment, silicone or some other elastomeric material substantially fills the interior within the dashed lines other than the components of the implantable device (e.g., there can be pockets within the dashed line in which no components and no silicone is located).

It is noted that FIGs. 1B and 1C are conceptual FIGs. presented for purposes of discussion. Commercial embodiments corresponding to these FIGs. can be different from that depicted in the figures.

Additional details of the plates, magnets, and housing made of elastomeric material will be described in greater detail below. First, however, additional functional details of the cochlear implant 100 will now be described.

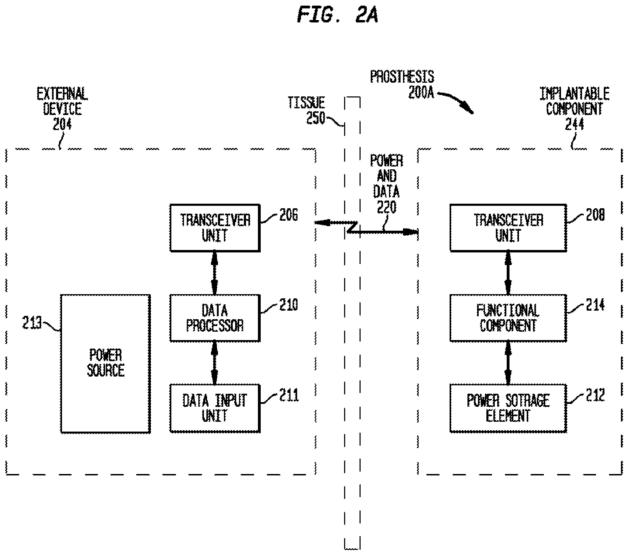

FIG. 2A is a functional block diagram of a prosthesis 200A in accordance with embodiments of the present invention. Prosthesis 200A comprises an implantable component 244 configured to be implanted beneath a recipient's skin or other tissue 250 and an external device 204. For example, implantable component 244 may be implantable component 100 of FIG. 1A, and external device may be the external device 142 of FIG. 1A. Similar to the embodiments described above with reference to FIG. 1A, implantable component 244 comprises a transceiver unit 208 which receives data and power from external device 204. External device 204 transmits power and data 220 via transceiver unit 206 to transceiver unit 208 via a magnetic induction data link 220. As used herein, the term receiver refers to any device or component configured to receive power and/or data such as the receiving portion of a transceiver or a separate component for receiving. The details of transmission of power and data to transceiver unit 208 are provided below. With regard to transceivers, it is noted at this time that while embodiments of the present invention may utilize transceivers, separate receivers and/or transmitters may be utilized as appropriate. This will be apparent in view of the description below.

Implantable component 244 may comprises a power storage element 212 and a functional component 214. Power storage element 212 is configured to store power received by transceiver unit 208, and to distribute power, as needed, to the elements of implantable component 244. Power storage element 212 may comprise, for example, a rechargeable battery 212. An example of a functional component may be a stimulator unit 120 as shown in FIG. 1B.

In certain embodiments, implantable component 244 may comprise a single unit having all components of the implantable component 244 disposed in a common housing. In other embodiments, implantable component 244 comprises a combination of several separate units communicating via wire or wireless connections. For example, power storage element 212 may be a separate unit enclosed in a hermetically sealed housing. The implantable magnet apparatus and plates associated therewith may be attached to or otherwise be a part of any of these units, and more than one of these units can include the magnet apparatus and plates according to the teachings detailed herein and/or variations thereof.

In the embodiment depicted in FIG. 2A, external device 204 includes a data processor 210 that receives data from data input unit 211 and processes the received data. The processed data from data processor 210 is transmitted by transceiver unit 206 to transceiver unit 208. In an exemplary embodiment, data processor 210 may be a sound processor, such as the sound processor of FIG. 1A for the cochlear implant thereof, and data input unit 211 may be a microphone of the external device.

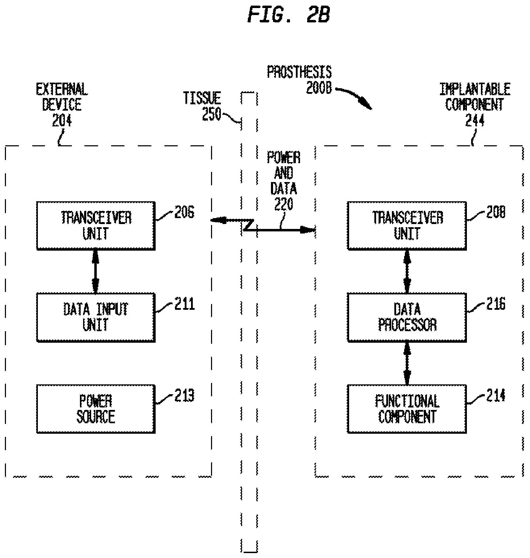

FIG. 2B presents an alternate embodiment of the prosthesis 200A of FIG. 2A, identified in FIG. 2B as prosthesis 200B. As may be seen from comparing FIG. 2A to FIG. 2B, the data processor can be located in the external device 204 or can be located in the implantable component 244. In some embodiments, both the external device 204 and the implantable component 244 can include a data processor.

As shown in FIGS. 2A and 2B, external device 204 can include a power source 213. Power from power source 213can be transmitted by transceiver unit 206 to transceiver unit 208 to provide power to the implantable component 244, as will be described in more detail below.

While not shown in FIGS. 2A and 2B, external device 204 and/or implantable component 244 include respective inductive communication components. These inductive communication components can be connected to transceiver unit 206 and transceiver unit 208, permitting power and data 220 to be transferred between the two units via magnetic induction.

As used herein, an inductive communication component includes both standard induction coils and inductive communication components configured to vary their effective coil areas.

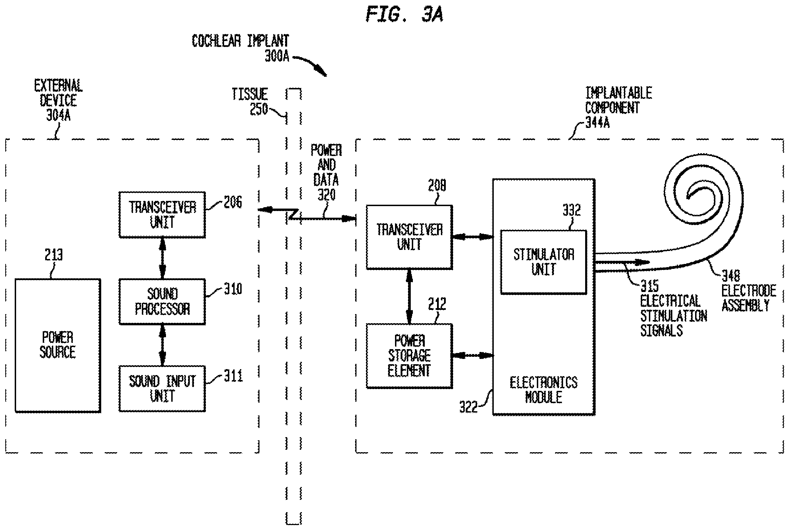

As noted above, prosthesis 200A of FIG. 2A may be a cochlear implant. In this regard, FIG. 3A provides additional details of an embodiment of FIG. 2A where prosthesis 200A is a cochlear implant. Specifically, FIG. 3A is a functional block diagram of a cochlear implant 300 in accordance with embodiments of the present invention.

It is noted that the components detailed in FIGS. 2A and 2B may be identical to the components detailed in FIG. 3A, and the components of 3A may be used in the embodiments depicted in FIGS. 2A and 2B.

Cochlear implant 300A comprises an implantable component 344A (e.g., implantable component 100 of FIG. 1) configured to be implanted beneath a recipient's skin or other tissue 250, and an external device 304A. External device 304A may be an external component such as external component 142 of FIG. 1.

Similar to the embodiments described above with reference to FIGS. 2A and 2B, implantable component 344A comprises a transceiver unit 208 (which may be the same transceiver unit used in FIGS. 2A and 2B) which receives data and power from external device 304A. External device 304A transmits data and/or power 320 to transceiver unit 208 via a magnetic induction data link. This can be done while charging module 202.

Implantable component 344A also comprises a power storage element 212, electronics module 322 (which may include components such as sound processor 126 and/or may include a stimulator unit 322 corresponding to stimulator unit 122 of FIG. 1B) and an electrode assembly 348 (which may include an array of electrode contacts 148 of FIG. 1A). Power storage element 212 is configured to store power received by transceiver unit 208, and to distribute power, as needed, to the elements of implantable component 344A.

As shown, electronics module 322 includes a stimulator unit 332. Electronics module 322 can also include one or more other functional components used to generate or control delivery of electrical stimulation signals 315 to the recipient. As described above with respect to FIG. 1A, electrode assembly 348 is inserted into the recipient's cochlea and is configured to deliver electrical stimulation signals 315 generated by stimulator unit 332 to the cochlea.

In the embodiment depicted in FIG. 3A, the external device 304A includes a sound processor 310 configured to convert sound signals received from sound input unit 311 (e.g., a microphone, an electrical input for an FM hearing system, etc.) into data signals. In an exemplary embodiment, the sound processor 310 corresponds to data processor 210 of FIG. 2A.

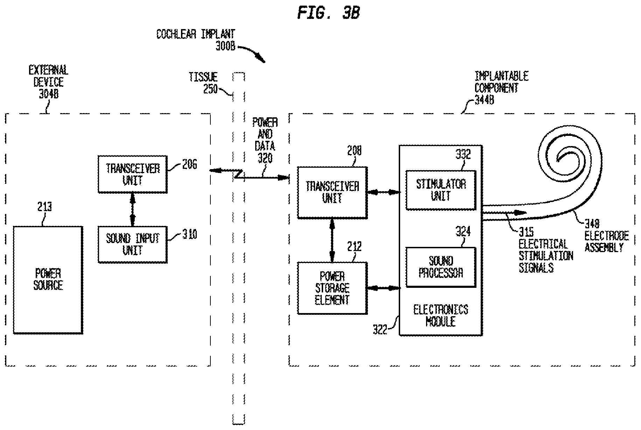

FIG. 3B presents an alternate embodiment of a cochlear implant 300B. The elements of cochlear implant 300B correspond to the elements of cochlear implant 300A except that external device 304B does not include sound processor 310. Instead, the implantable component 344B includes a sound processor 324, which may correspond to sound processor 310 of FIG. 3A.

As will be described in more detail below, while not shown in the figures, external device 304A/304B and/or implantable component 344A/344B include respective inductive communication components.

FIGS. 3A and 3B illustrate that external device 304A/304B can include a power source 213, which may be the same as power source 213 depicted in FIG. 2A. Power from power source 213 can be transmitted by transceiver unit 306 to transceiver unit 308 to provide power to the implantable component 344A/344B, as will be detailed below. FIGS. 3A and 3B further detail that the implantable component 344A/344B can include a power storage element 212 that stores power received by the implantable component 344 from power source 213. Power storage element 212 may be the same as power storage element 212 of FIG. 2A.

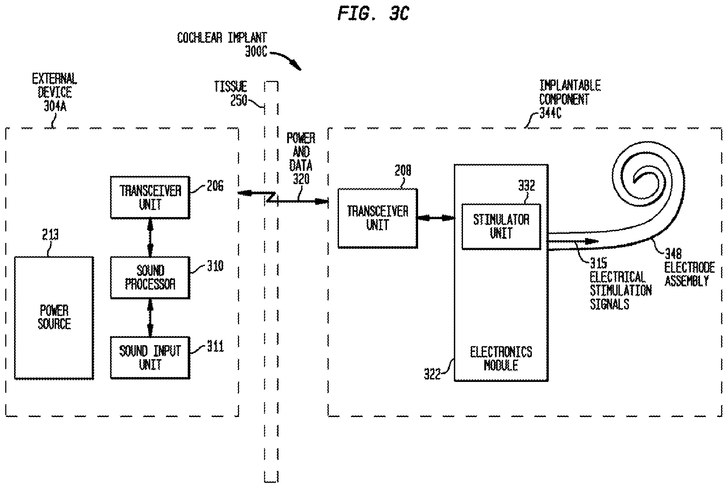

In contrast to the embodiments of FIGS. 3A and 3B, as depicted in FIG. 3C, an embodiment of the present invention of a cochlear implant 300C includes an implantable component 344C that does not include a power storage element 212. In the embodiment of FIG.3C, sufficient power is supplied by external device 304A/304B in real time to power implantable component 344C without storing power in a power storage element. In FIG. 3C, all of the elements are the same as FIG. 3A except for the absence of power storage element 212.

Some of the components of FIGS. 3A-3C will now be described in greater detail.

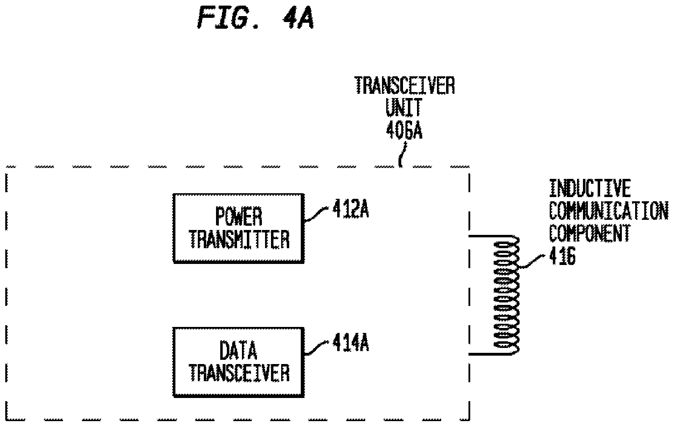

FIG. 4A is a simplified schematic diagram of a transceiver unit 406A in accordance with an embodiment of the present invention. An exemplary transceiver unit 406A may correspond to transceiver unit 206 of FIGS. 2A-3C. As shown, transceiver unit 406A includes a power transmitter 412 a, a data transceiver 414A and an inductive communication component 416.

In an exemplary embodiment, as will be described in more detail below, inductive communication component 416 comprises one or more wire antenna coils (depending on the embodiment) comprised of multiple turns of electrically insulated single-strand or multi-strand platinum or gold wire (thus corresponding to coil 137 of FIG. 1B). Power transmitter 412A comprises circuit components that inductively transmit power from a power source, such as power source 213, via an inductive communication component 416 to implantable component 344A/B/C (FIGS. 3A-3C). Data transceiver 414A comprises circuit components that cooperate to output data for transmission to implantable component 344A/B/C (FIGS. 3A-3C). Transceiver unit 406A can receive inductively transmitted data from one or more other components of cochlear implant 300A/B/C, such as telemetry or the like from implantable component 344A (FIG. 3A).

Transceiver unit 406A can be included in a device that includes any number of components which transmit data to implantable component 334A/B/C. For example, the transceiver unit 406A may be included in a behind-the-ear (BTE) device having one or more of a microphone or sound processor therein, an in-the-ear device, etc.

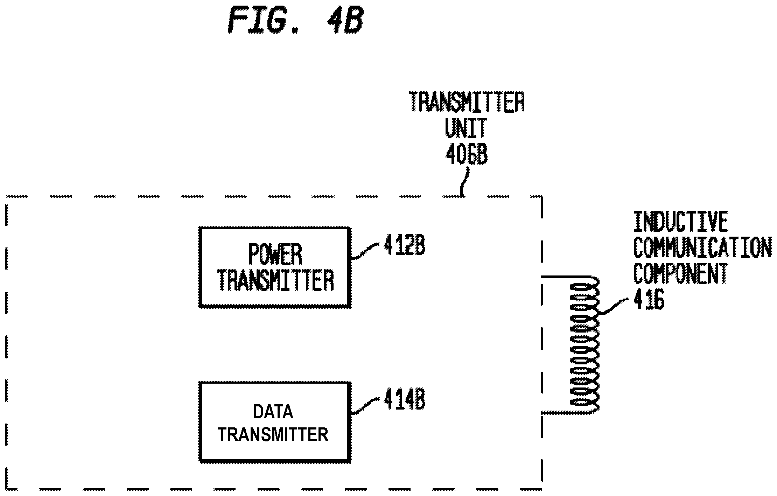

FIG. 4B depicts a transmitter unit 406B, which is identical to transceiver unit 406A, except that it includes a power transmitter 412B and a data transmitter 414B.

It is noted that for ease of description, power transmitter 412A and data transceiver 414A / data transmitter 414B are shown separate. However, it should be appreciated that in certain embodiments, at least some of the components of the two devices may be combined into a single device.

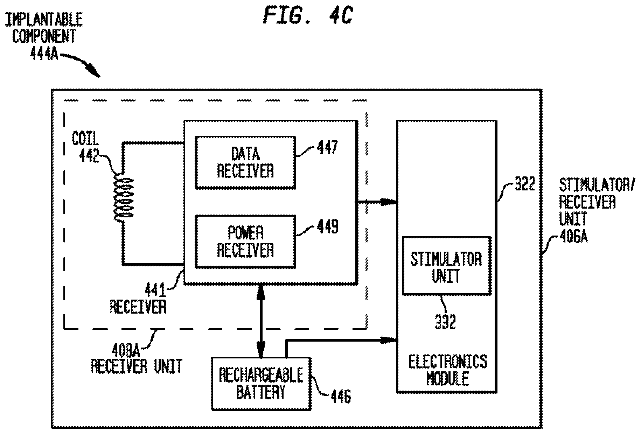

FIG. 4C is a simplified schematic diagram of one embodiment of an implantable component 444A that corresponds to implantable component 344A of FIG. 3A, except that transceiver unit 208 is a receiver unit. In this regard, implantable component 444A comprises a receiver unit 408A, a power storage element, shown as rechargeable battery 446, and electronics module 322, corresponding to electronics module 322 of FIG. 3A. Receiver unit 408A includes an inductance coil 442 connected to receiver 441. Receiver 441 comprises circuit components which receive via an inductive communication component corresponding to an inductance coil 442 inductively transmitted data and power from other components of cochlear implant 300A/B/C, such as from external device 304A/B. The components for receiving data and power are shown in FIG. 4C as data receiver 447 and power receiver 449. For ease of description, data receiver 447 and power receiver 449 are shown separate. However, it should be appreciated that in certain embodiments, at least some of the components of these receivers may be combined into one component.

In the illustrative embodiments of the present invention, receiver unit 408A and transceiver unit 406A (or transmitter unit 406B) establish a transcutaneous communication link over which data and power is transferred from transceiver unit 406A (or transmitter unit 406B), to implantable component 444A. As shown, the transcutaneous communication link comprises a magnetic induction link formed by an inductance communication component system that includes inductive communication component 416 and coil 442.

The transcutaneous communication link established by receiver unit 408A and transceiver unit 406A (or whatever other viable component can so establish such a link), in an exemplary embodiment, may use time interleaving of power and data on a single radio frequency (RF) channel or band to transmit the power and data to implantable component 444A. A method of time interleaving power according to an exemplary embodiment uses successive time frames, each having a time length and each divided into two or more time slots. Within each frame, one or more time slots are allocated to power, while one or more time slots are allocated to data. In an exemplary embodiment, the data modulates the RF carrier or signal containing power. In an exemplary embodiment, transceiver unit 406A and transmitter unit 406B are configured to transmit data and power, respectively, to an implantable component, such as implantable component 344A, within their allocated time slots within each frame.

The power received by receiver unit 408A can be provided to rechargeable battery 446 for storage. The power received by receiver unit 408A can also be provided for distribution, as desired, to elements of implantable component 444A. As shown, electronics module 322 includes stimulator unit 332, which in an exemplary embodiment corresponds to stimulator unit 322 of FIGS. 3A-3C, and can also include one or more other functional components used to generate or control delivery of electrical stimulation signals to the recipient.

In an embodiment, implantable component 444A comprises a receiver unit 408A, rechargeable battery 446 and electronics module 322 integrated in a single implantable housing, referred to as stimulator/receiver unit 406A. It would be appreciated that in alternative embodiments, implantable component 344 may comprise a combination of several separate units communicating via wire or wireless connections.

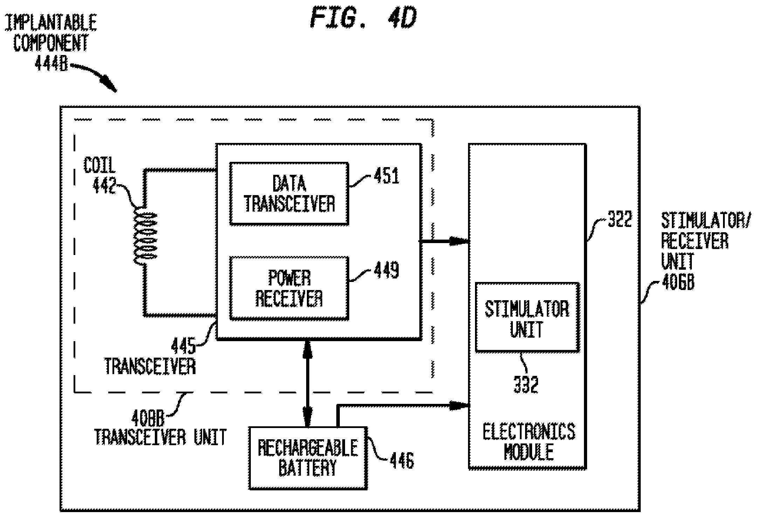

FIG. 4D is a simplified schematic diagram of an alternate embodiment of an implantable component 444B. Implantable component 444B is identical to implantable component 444A of FIG. 4C, except that instead of receiver unit 408A, it includes transceiver unit 408B. Transceiver unit 408B includes transceiver 445 (as opposed to receiver 441 in FIG. 4C). Transceiver unit 445 includes data transceiver 451 (as opposed to data receiver 447 in FIG. 4C).

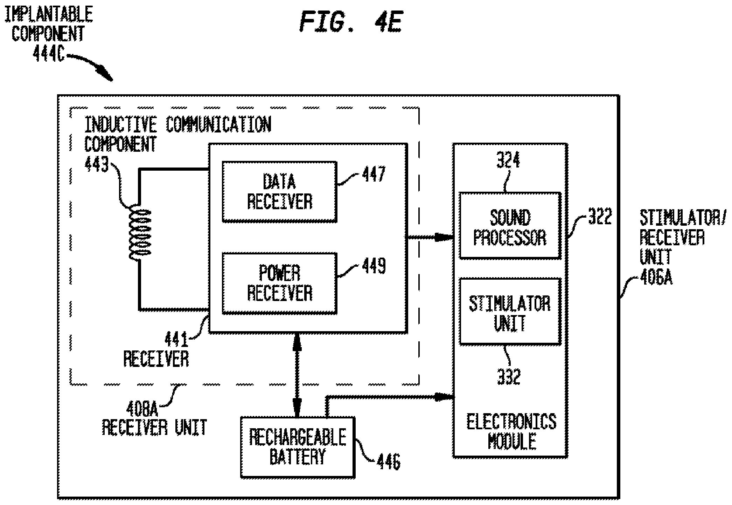

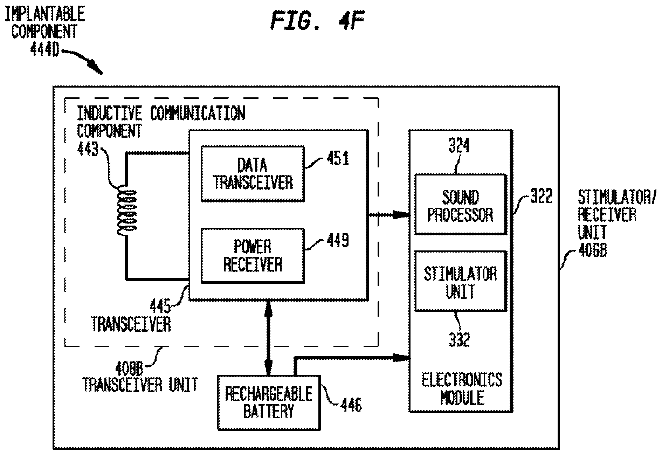

FIGS. 4E and 4F depict alternate embodiments of the implantable components 444A and 444B depicted in FIGS. 4C and 4D, respectively. In FIGS. 4E and 4F, instead of coil 442, implantable components 444C and 444D (FIGS. 4E and 4F, respectively) include inductive communication component 443. Inductive communication component 443 is configured to vary the effective coil area of the component, and may be used in cochlear implants where the exterior device 304A/B does not include a communication component configured to vary the effective coil area (i.e., the exterior device utilizes a standard inductance coil). In other respects, the implantable components 444C and 444D are substantially the same as implantable components 444A and 444B. Note that in the embodiments depicted in FIGS. 4E and 4F, the implantable components 444C and 444D are depicted as including a sound processor 342. In other embodiments, the implantable components 444C and 444D may not include a sound processor 342.

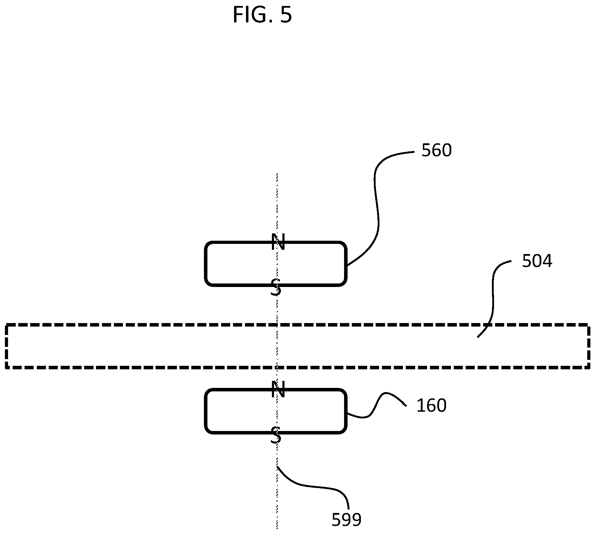

FIG. 5 represents a high level conceptual exemplary magnetic coupling arrangement according to an exemplary embodiment, except that the magnet apparatus160 is a disk shaped magnet in a disk shaped housing, and is presented for conceptual purposes. Specifically, FIG. 5 presents the magnet apparatus 160 of the implantable component 100 having a longitudinal axis 599 aligned with the magnet 560 of the external device 142, along with a functional representation of the tissue 504 of the recipient located between the two components. All other components of the external device and implantable component are not shown for purposes of clarity. As can be seen, the magnet apparatus 160 as a north-south polar axis aligned with the longitudinal axis 599, and magnet apparatus 560 also has a north-south polar axis aligned with the longitudinal axis of that magnet apparatus. In the exemplary embodiment, owing to the arrangements of the magnets, the resulting magnetic field aligns the magnets such that the longitudinal axes of the magnets are aligned. In an exemplary embodiment, because the various coils of the devices are aligned with the various longitudinal axes of the magnets, the alignment of the magnets aligns the coils.

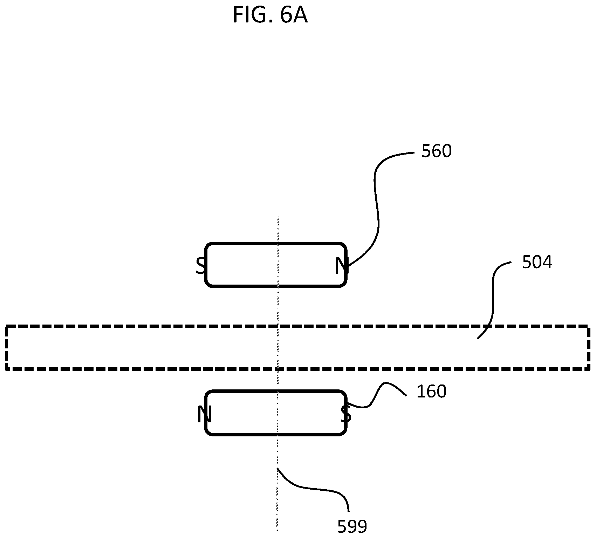

FIG. 6A presents an alternative embodiment, where the magnet apparatus 160 of the implantable component 100 has a north-south axis aligned with the lateral axis of the magnet apparatus, as can be seen. In this exemplary embodiment, the magnet 560 also has a north-south axis also aligned with the lateral axis of that magnet.

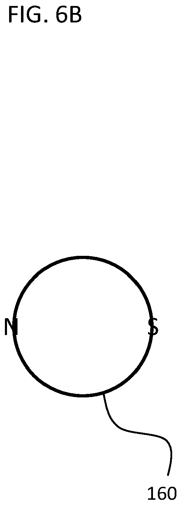

The magnet apparatus 160 and 5690 are disk shaped / has the form of a short cylinder. FIG. 6B presents a top view of magnet apparatus 160, showing that the outer profile is circular, consistent with the fact that magnet 160 is a disk / short cylinder. With respect to the embodiment of figure 5, the "N" (North Pole of the magnet) would be in the center of the circle representing magnet apparatus 160. The magnet of the external device 142 can also have such a form. That said, in an alternative embodiment, the magnets can have another configuration (e.g., a plate magnet, a bar magnet, etc.). Moreover, in an alternative embodiment, two or more magnets can be used in the implantable device and/or in the external device. The magnets could be located outboard of the coil. Any arrangement of magnet(s) of any configuration that can have utilitarian value according to the teachings detailed herein and/or variations thereof can be utilized in at least some embodiments.

In some embodiments, as seen above, there is utility in using a magnet to retain the external coil. This means that there can be a magnet that is present in the implant during MRI which imparts significant torque to the magnet which can in turn cause discomfort or device damage, e.g., magnet dislodgement.

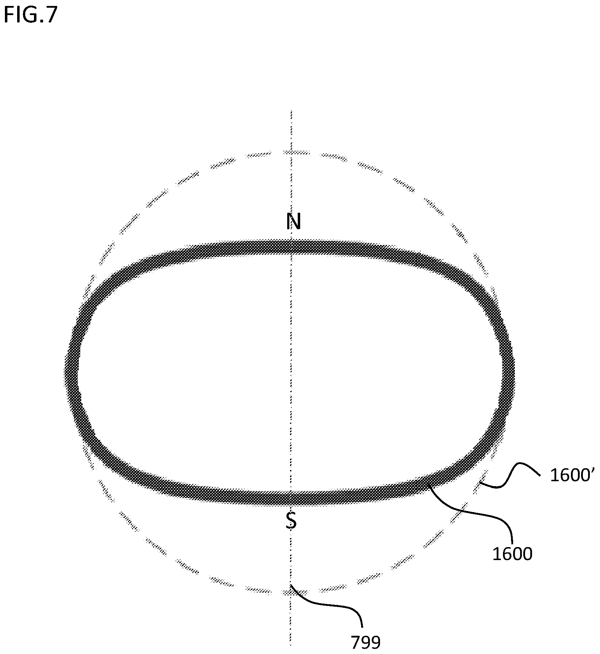

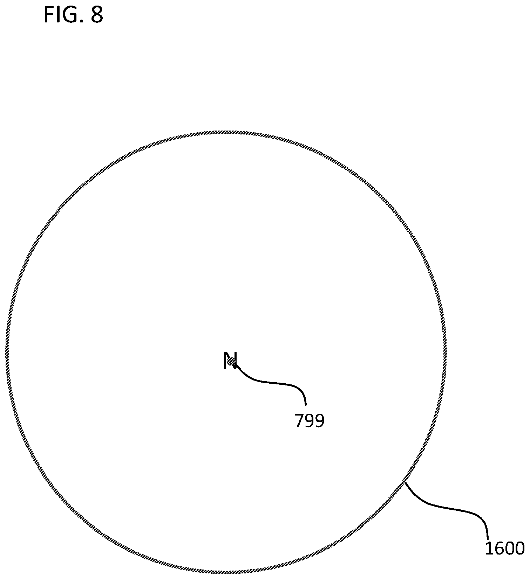

Conversely, an embodiment can have the poles aligned in the orientation of FIG. 5, and have the magnet apparatus rotate in the plane of axis 599 / rotate about an axis that is normal to axis 599 (i.e., one extending into and out of the page of FIG. 5). FIG. 7 depicts such an exemplary embodiment, where there is a modified spherical magnet apparatus 1600 that is able to rotate in the MRI's magnetic field, but has less thickness and has more retention strength than a diametrically opposed arrangement. Here, the magnet apparatus 1600 can be compared to the unmodified sphere 1600', which, in some embodiments, is a 7 mm diameter sphere, and thus the long diameter of the modified sphere shape is also 7 mm, although it is noted that in other embodiments, different dimensions can be utilized. By utilizing a magnet apparatus that is a modified spherical shape, a shorter diameter can be implemented with respect to the dimension extending normal from the surface of the bone, thus reducing the thickness of the implant. FIG. 8 depicts a top view of the magnet apparatus 1600 (i.e., looking down onto the magnet / looking in the direction normal to the skull surface if the magnet was implanted in a recipient). As can be seen, the magnet apparatus has maximum outer profile that is circular in this embodiment.

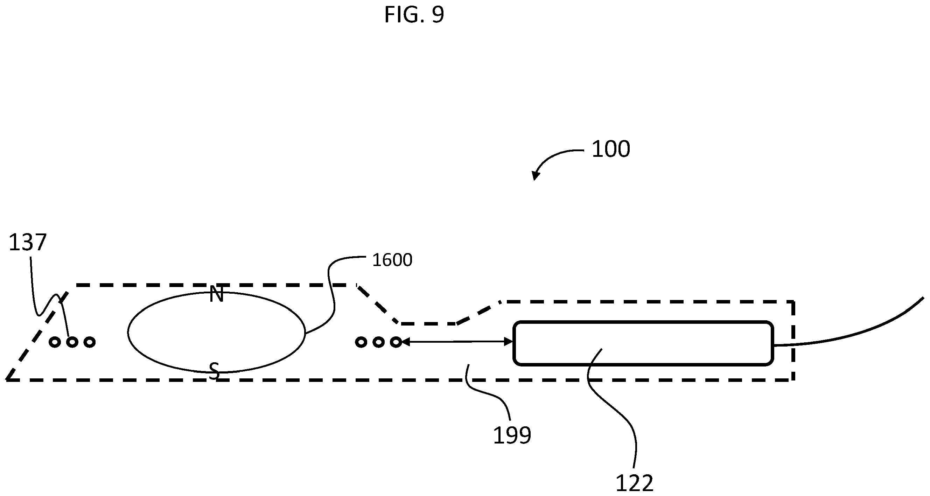

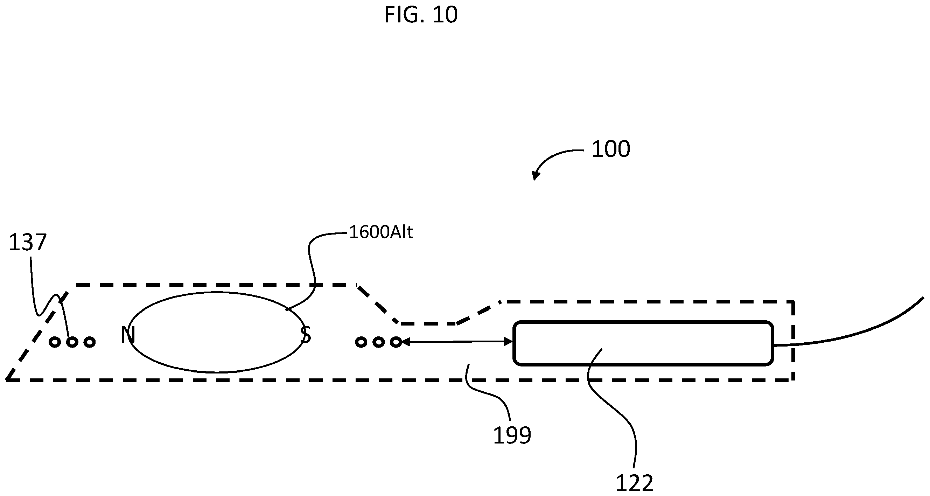

Figure 9 depicts magnet apparatus 1600 located in the implant 100 during normal operation / when the recipient and/or the implant 100 is not subjected to an MRI magnetic field according to at least some of the magnetic fields detailed herein. In this exemplary embodiment, the north south polarity of the magnet apparatus 1600 is analogous to that of figure 5. This provides a retention force that retains the external component to the recipient via a force that is greater than that which would be the case with respect to the arrangement depicted in figure 6A, or even FIG. 10, where the volume of the magnet apparatus 1600Alt of the implant would be larger than the implant magnet of figure 6A, and comparable to that of FIG. 9, but the retention force would be lower than that of FIG. 9, such as by at least 5, 10, 15, 20, 25, 30, 35, 40, 45, 50, 55, 60, 65, 70, 75, or 80 % or more, or any value or range of values therebetween in 1% increments (7, 22, 8 to 56 percent, etc.), all other things being equal. (These numbers can be applicable to the arrangement of FIG. 6A as well, where the magnetic volume of FIG. 6A is the same as FIG. 9.) If utilitarian, the magnet design of FIG. 9 could be made smaller to achieve a given retention, such as that achieved by that of FIG. 6A (in embodiments where the figures are to scale). That is, the embodiment of FIG 6A may provide less retention force relative to that which would be the case if the alignment was that of FIG. 5 (at least with respect to the implanted magnet), all other things being equal (same size and/or volume magnet, same material, same magnetization quality, etc.), and thus the size could be smaller if similar retention qualities were desired.

Briefly, the magnet can be located outboard of the coil in some embodiments. Any arrangement of magnet(s) of any configuration that can have utilitarian value according to the teachings detailed herein and/or variations thereof can be utilized in at least some embodiments.

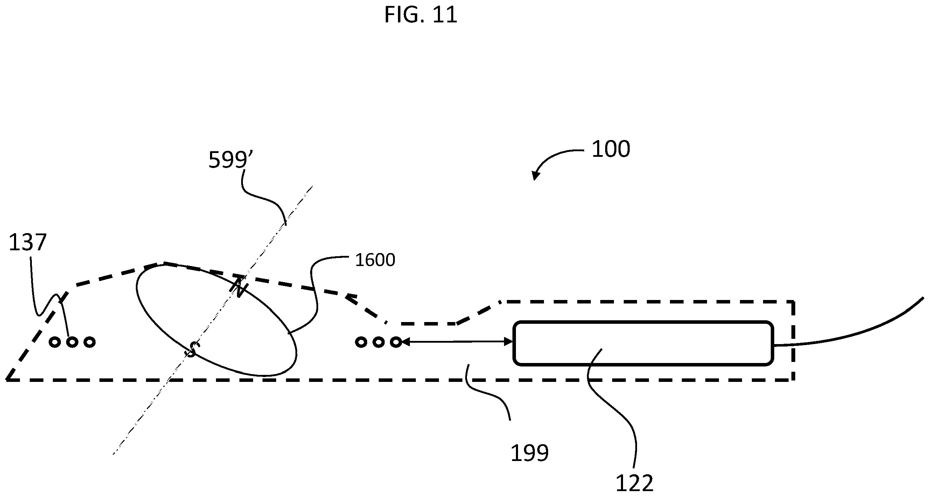

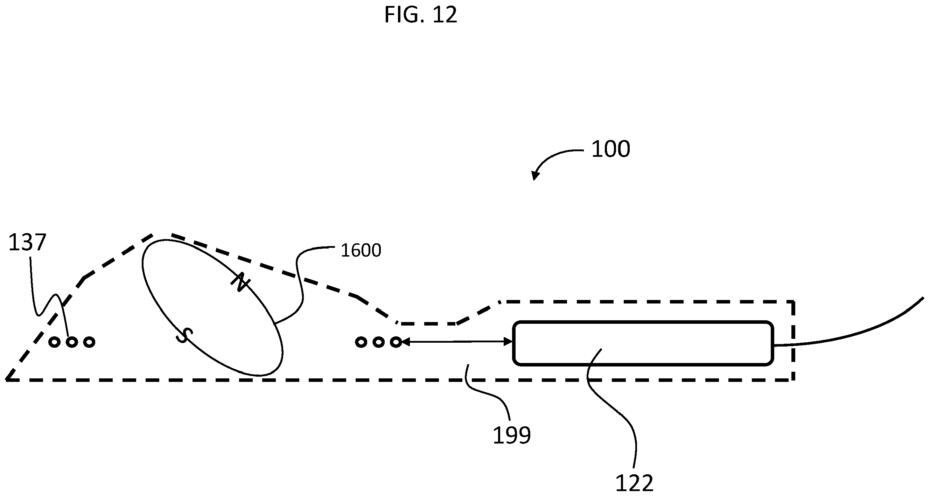

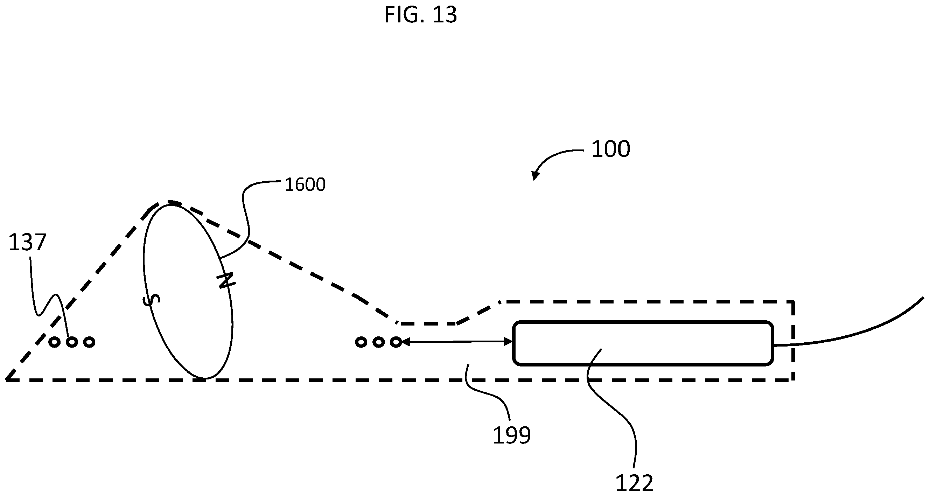

FIGs. 11-14 depict exemplary scenarios of operation of the implant when the implant is exposed to a magnetic field of an MRI machine that is aligned in a direction that is not aligned with the poles of the magnet apparatus as arranged in FIG. 9. (e.g., offset by less than, greater than or about equal to 20, 25, 30, 35, 40, 45, 50, 55, 60, 65, 70, 75, 80, 85, or 90 degrees or any value or range of values therebetween in 1 degree increments). Here, the implant 100 is located in the head of the recipient between the skull and the skin and is thus held generally stationary relative to the head due to the skin and/or to bone fixtures / bone screws, etc. A relatively strong external magnetic field is applied to the magnet apparatus 1600 while the magnet apparatus 1600, and thus the implantable component 100, is implanted in a recipient at a location in the recipient corresponding to that which is where the implantable component would be implanted for normal use thereof (e.g., the magnet apparatus 1600 can be located above the mastoid bone of the recipient and beneath the skin of the recipient for a cochlear implant). In an exemplary embodiment, the external magnetic field is that which results from an MRI machine during MRI imaging of the recipient's head or body (with the implantable component 100, and thus the magnet apparatus 1600, implanted therein), where the magnetic field generated by the MRI machine interacts with the magnetic field of the magnet apparatus 1600 to impart a significant torque onto the magnet apparatus 1600. In an exemplary embodiment, the torque is up to 0.38 Newton meters (if there is more magnet material / a stronger magnet material is used, this can be higher), if the implant were to resist the torque perfectly, and the MRI machine applies a magnetic field such that the implanted magnet apparatus 1600 is subjected to a 3 T magnetic field. The explanation below refers to a 3T magnetic field, but is applicable to any applied field, such as by way of example only and not by way of example, any of the fields disclosed herein.

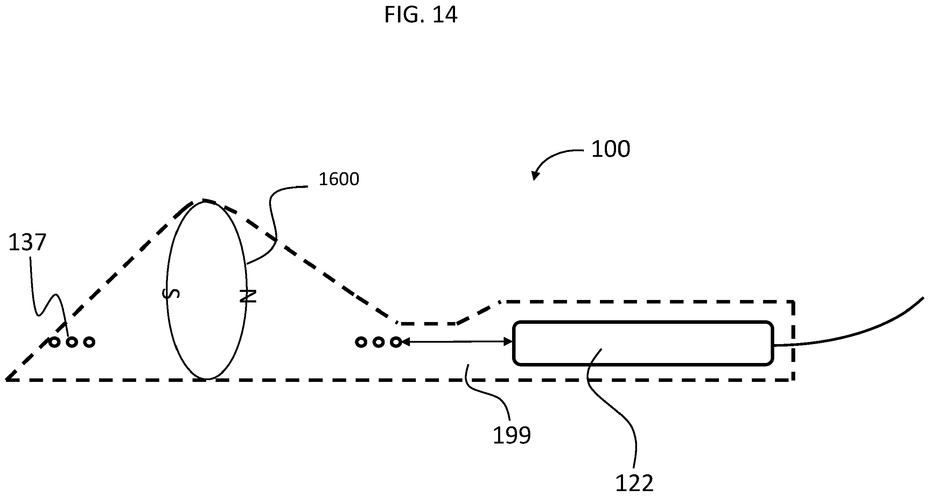

In at least some exemplary embodiments, the magnet apparatus is free to rotate to the 90° position seen in figure 14, and beyond. In at least some exemplary embodiments, the magnet apparatus could become "stuck" or otherwise remain in the 90° position after the magnetic field has been removed. In an exemplary embodiment, the implantable component is configured so that a person could move the magnet apparatus by hand, indirectly, by pushing on one side of the magnet apparatus or more accurately, by pushing on the skin on one side of the magnet apparatus, so that it rotates back to the at rest position. In an exemplary embodiment, the magnet apparatus could be massaged back to its original position. In at least some exemplary embodiments, the implantable component can be configured to prevent the magnet apparatus from rotating more than 90°, or even from rotating more than 85° or 80°, or any value that can be utilitarian. Some exemplary embodiments that prevent such rotation are described below. The point is, in some embodiments, it is possible for the magnet to become stuck at the rotated position, and embodiments are configured to noninvasively return the magnet to its at rest position. That said, some embodiments can be configured to prevent rotation to values that would cause the magnet to become stuck and/or that could complicate returning the magnet to its at rest position, even with the above-noted hand massaging or the like. In this regard, in an exemplary embodiment, there can be utilitarian value with respect to preventing the magnet from rotating more than 90° because such could result in the polarity of the magnetic field that is generated by the magnet apparatus relative to the surface of the skin being reversed, because the magnet apparatus has basically been flipped upside down so that, with respect to the figures, the north pole faces towards the bone as opposed to the skin. By preventing the magnet from rotating more than 90°, for example, the shortest route back to the at rest position will always results in the proper polarity orientation. That said, in some embodiments, the rotation can be permitted to extend beyond 90°, but limited to an amount where a healthcare professional or the like can still massaged the magnet apparatus back to its at rest position. For example, the magnet apparatus can be prevented from rotating more than 120°, and by some semi-trained tactile inspection, the caregiver could recognize that the magnet resists further movement in one direction, but permits movement in the other direction, thus indicating that the other direction is the direction that will result in the magnet returning to the at rest position with the desired polarity orientation.

Alternatively, and/or in addition to this, the healthcare professional can apply a magnet with a known north south pole or could apply the external component against the skin of the recipient, to ascertain the orientation of the polarity of the magnet. A healthcare professional who is at least semi-trained with respect to the possible magnet placement scenarios could recognize which way the magnet should be rotated to render the polarity orientation utilitarian vis-à-vis holding the external component to the recipient. In an exemplary embodiment, the healthcare professional could access the instructions for providing the recipient in MRI and follow those instructions to return the magnet apparatus to its at rest position with the desire polarity.

Still further, in an exemplary embodiment, a strong magnet could be used to rotate the magnet apparatus back to its proper magnetic field orientation. This strong magnet could be a magnet that is supplied to MRI professionals for this purpose. The strong magnet can be located in an apparatus that self-centers the strong magnet proximate the implanted magnet apparatus. That said, in an alternative embodiment, the MRI machine could be utilized to move the magnet. In an exemplary embodiment, the healthcare professional could instruct the recipient to move his or her head to a certain angle that is not normal when taking MRI scans but which will have utilitarian value with respect to imparting a torque onto the magnet apparatus that will cause the magnet apparatus to rotate at least towards its at rest and proper polarity orientation. Alternatively, or in addition to this, an electromagnet device can be provided, where, for example, an electrical current is used to generate a magnetic field, that forces the magnet apparatus to rotate back to the desired polarity alignment.

Also, in at least some exemplary embodiments, the external component can be configured so that the polarity direction of the magnet of the external component can be reversed with relative ease, or at least without breaking or otherwise destroying the external component. In this regard, the magnet of the external component can be removable and can be of a configuration that will permit the magnet of the external component to be flipped over so that the polarity of the magnetic field would be opposite that which was previously the case, so as to accommodate the implanted magnet which now has polarity direction that is opposite that which was previously the case. In an exemplary embodiment, the external component can be configured to be flipped completely upside down without any changes thereto to accommodate the new polarity orientation. That is, in an exemplary embodiment, the headpiece of the external component can be configured with two skin interfacing sides, opposite one another, so that the magnet located therein, regardless of the orientation of the magnetic field of the implant and/or the external component, will be attracted to the magnet of the implant.

In some embodiments, the magnet apparatus has a shape and the implant is configured such that with some massaging of the skin by a healthcare professional, the magnet apparatus can be flipped upside down without exposure to a magnetic field, thus returning the magnet apparatus to its original orientation.

Still, in at least some exemplary embodiments, the implant is configured to prevent rotation to 90° and/or beyond 90°. More on this below.

In an exemplary embodiment, the implantable device is configured to avoid a top-dead-center position of the magnet apparatus, such as that shown in FIG. 14.

In an exemplary embodiment, the magnetic field is generated by an MRI machine. That said, in some embodiments, the magnetic field is generated by an open MRI. The teachings detailed herein are applicable to any MRI that imparts a magnetic field onto the magnet of the magnet apparatus 1600 that imparts a torque onto the magnet.

The magnet apparatus 1600 rotates in the plane of the pages / about an axis that is normal to the plane of the pages, to better align with the magnetic field generated by the MRI. The elastomeric material 199 stretches or otherwise deforms to permit the magnet apparatus 1600 to rotate, while providing some resistance there against. In this exemplary embodiment, the elastomeric material provides a modicum of resistance to rotation of the magnet, which resistance will typically prevent the magnet from rotating during normal operation, such as when exposed to a low strength magnetic field according to that which is generated by the external component and/or when subjected to low physical forces, but will enable the magnet to rotate when the magnet is subjected to a magnetic field of an MRI machine, for example, such as one or more the magnetic fields detailed herein, which can be greater than less than or equal to 1, 1.5, 2, 2.5, 3, 3.5, 4, 4.5, 5, 6, 7, 8, 9, 10 T or more magnetic fields. By permitting the magnet to rotate, the magnet can align itself with the magnetic field of the MRI and thus reduce, including eliminate, any demagnetization which may occur and/or reduce (including eliminate) the amount of force that is felt by the recipient / reduce (including eliminate) the amount of discomfort felt by the recipient. Further, physical damage to the implantable component can be prevented or otherwise the likelihood of physical damage can be reduced relative to that which would otherwise be the case if the magnet could not rotate relative to the implantable component, all other things being equal.

As can be seen from the figures, in an exemplary embodiment, the implantable component 100 is configured such that the elastomeric material deforms due to rotation of the magnet apparatus 1600 as a result of the torque applied thereto due to the 3 T magnetic field. As can be seen, the magnet apparatus 1600 rotates such that its longitudinal axis moves from its normal position (the position where the magnet is located in the absence of an external magnetic field, where the longitudinal axis 599 of the magnet apparatus 1600 is at least generally normal to the bottom base of the implantable component), while also stabilizing and holding the magnet apparatus within the implant. Owing to the rotation of the magnet 1600, the magnet 1600 is tilted relative to the base of the implant. FIG. 11 shows the longitudinal axis 599 of the magnet 1600 shifted from its normal position (599'). In an exemplary embodiment, shift from the normal position in degrees is greater than, less than, or about equal to 5.0, 6.0, 7.0, 8.0, 9.0, 10.0, 11.0, 12.0, 13.0, 14.0, 15.0, 16.0, 17.0, 18.0, 19.0, 20.0, 21.0, 22.0, 23.0, 24.0, 25.0, 26.0, 27.0, 28.0, 29.0, 30.0, 31.0, 32.0, 33.0, 34.0, 35, 40, 45, 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 110, 115, 120, 125, 130 or more, or any value or range of values therebetween in about 0.1 increments (e.g., about 49.3 to about 58.1 degrees, 67.3 degrees, etc.).

In an exemplary embodiment, the implantable medical device includes a support body that includes a monolithic portion made of silicone (the portion can make up the entirety of the silicone body 199, or can be a portion of the silicone body) that at least partially envelops the magnet apparatus and positions the magnet apparatus such that the magnet apparatus is biased in a direction such that the long axis is generally parallel (which includes parallel) to a base of the device. In an exemplary embodiment, the support body includes a monolithic portion made of silicone that at least partially envelops the magnet apparatus and elastically deforms to enable the magnet apparatus to rotate about an axis parallel to the base / in a plane normal to the base of the device at least 30, 35, 40, 45, 50, 55, 60, 65, 70, 75, 80, 85, or 90 degrees, or any value or range of values therebetween in 1 degree increments from a relaxed orientation when subjected to a magnetic field of at least 1, 1.5, 2, 2.5, 3, 3.5 4, 4.5, 5, 6, or 7 T. In an exemplary embodiment, the implant is configured such that the change in thickness occurs freely. Conversely, as will be detailed below, in some embodiments, instead of the device being configured so that the change in thickness occurs freely, a plate or some other structure can be located between the silicone of the implant body and the magnet apparatus.

In some embodiments, the variation of the resistance torque can be within a given percentage over a given range of rotations. By way of example only and not by way of limitation, the resistance torque might increase until the magnet has rotated 10° from its at rest position, and then might remain relatively steady until the magnet has rotated about 40 or 50° from its at rest position, and then might increase again, while in other embodiments, the resistance torque might decrease after that amount of rotation. In an exemplary embodiment, the resistance torque remains within 1, 2, 3, 4, 5, 6, 7, 8, 9, 10, 11, 12, 13, 14, 15, 16, 17, 18, 19, 20, 25, 30, 35, 40, 45, or 50% of the lowest value experienced within a given range of the aforementioned ranges of rotation, with a given range can be any range of values within the above-noted possible rotation regimes in 0.1° increments.

In an exemplary embodiment, the resistance to rotation can increase linearly or exponentially and/or can decrease linearly or exponentially and/or combination thereof. In some embodiments, the resistance to rotation can increase linearly and then increase exponentially to tail off to a flat line, and then can decrease exponentially and then decrease linearly.

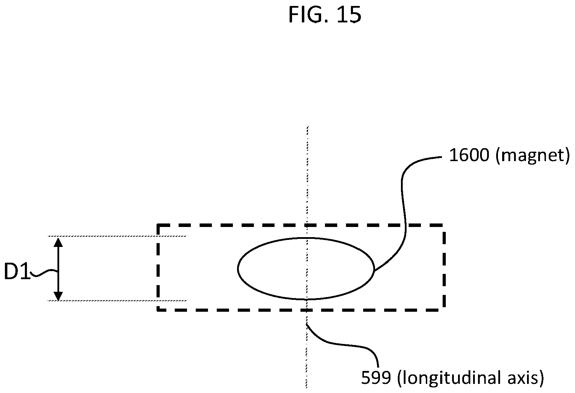

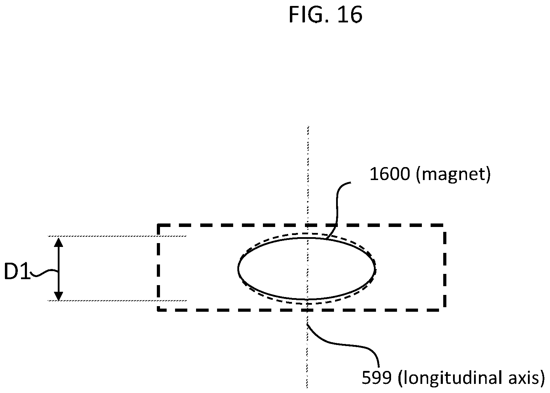

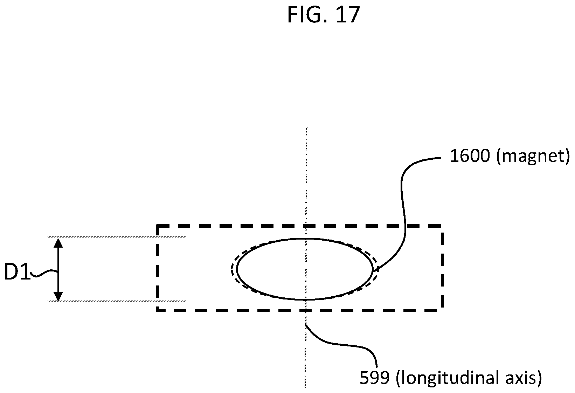

FIG. 15 presents a portion of the view of FIG. 1C (side view of FIG. 1B), showing the magnet apparatus 1600 located in a body of elastomeric material. All other components are removed for purposes of clarity. FIG. 15 depicts an rectangular-shaped dashed line structure, which conceptually represents a volume that is generally filled with an elastomeric material, such as silicone, thus conceptually representing the apparatus made from elastomeric material 199 of FIG. 1B. In an exemplary embodiment, the space is basically filled with silicone. That said, in an alternate embodiment, there are locations where there is no elastomeric material. FIGs. 16 and 17 present some examples, where the volume between the magnet apparatus 160 and the adjacent dashed lines is devoid of silicone. (Note that these views represent only the sides of the magnet apparatus and elastomeric apparatus with respect to the cross-section taken along the longitudinal axis of the implantable device 100 - the elastomeric material can be closer to the magnet apparatus on the lateral sides / away from the longitudinal axis of the implantable device 100, thus still maintaining the position of the magnet apparatus 160.)

In FIG. 15, D1 can be the short axis / minimum diameter of the magnetic apparatus.

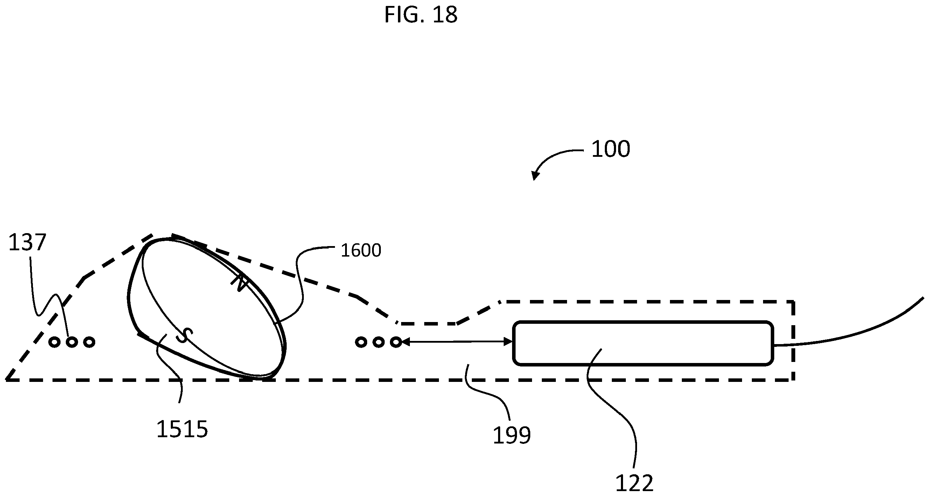

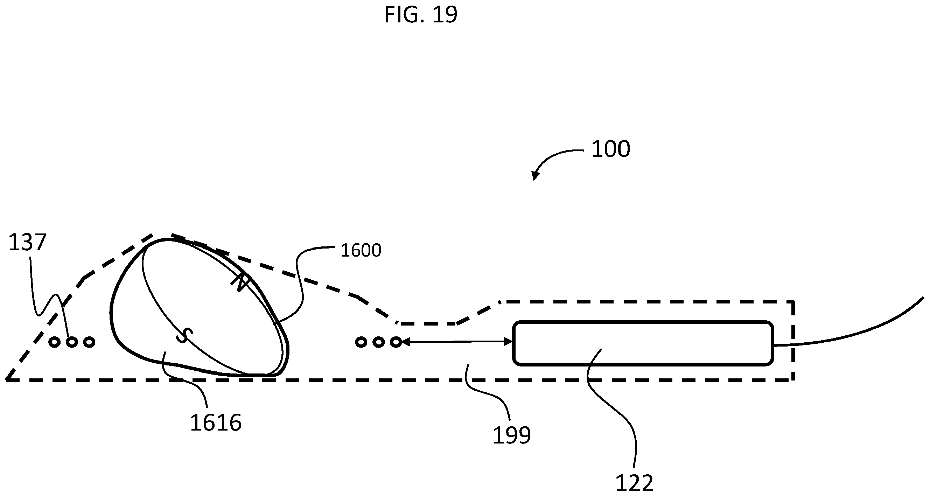

In an exemplary embodiment, the elastomeric material surrounding the magnet apparatus holds the magnet apparatus in place. In the embodiment of FIG. 15, the elastomeric material is in contact with essentially 100% of implantable thantable component 100 prior to exposure thereto to an MRI magnetic field, which could, in some embodiments, results in a condition during the exposure and/or after the exposure when the magnet returns to its normal position (that of FIG. 9) / at rest position where these values are different from that precedent. In this regard, figures 18 and 19 depict voids 1515 and 1616 respectively, that result from rotation of the magnet when exposed to a magnetic field with the implantable component restrained in the head of the recipient / by the head of the recipient. This is compared to the embodiments of figures 11-14, where no void is created / no substantive void is created, as a result of rotation by the magnet apparatus 1600. Thus, in an exemplary embodiment, the elastomeric material is in direct contact with a majority of the surface area of the magnet apparatus. (This does not mean that it is in direct contact with the magnet. In some embodiments, the magnet apparatus is a magnet that is housed in a housing of a biocompatible material, such as, for example, titanium or ceramic. In an exemplary embodiment, the magnet material is clad in a biocompatible material, while in other embodiments, the magnet material is supported within the housing of biocompatible material. It is the magnet apparatus, and thus in these embodiments, for example, the housing, that is in direct contact with the elastomeric material. In some embodiments, the magnet of the magnet apparatus can be configured as a casing that encases the magnet. Any disclosure herein of a magnet apparatus corresponds to a disclosure of a magnet without these barrier features as well as a magnet that includes these barrier features, and any disclosure herein of a magnet corresponds to a disclosure of a magnet with or without these barrier features.)

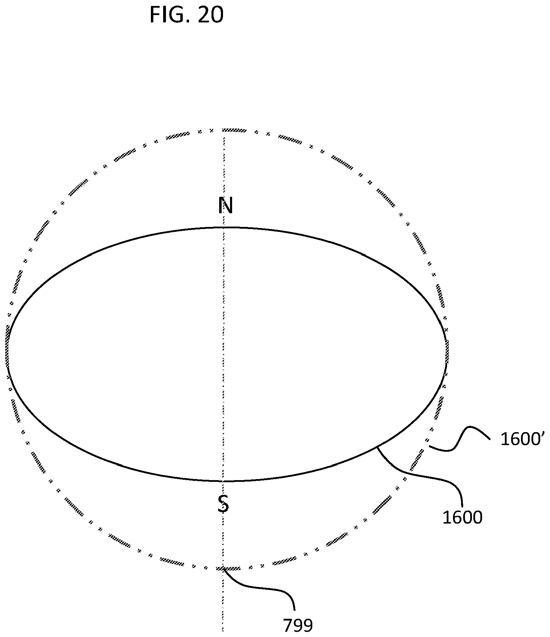

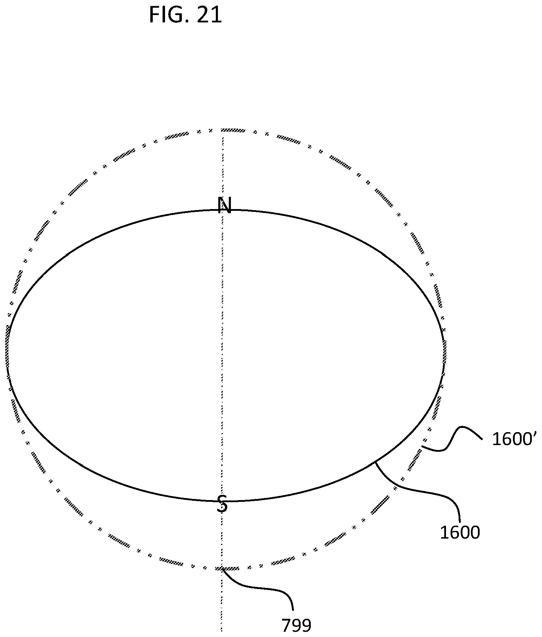

FIGs. 20-22 present exemplary embodiments in some alternate configurations of the magnet apparatus 1600 with reference to a perfect sphere 1600'. All of these views are side views, and when viewed from the top, a circular configuration would be seen. That said, in some alternate embodiments, the geometry of the magnet apparatus 1600 can also be such that the modified sphere shape is seen from the top view as well. Any arrangement that can enable the teachings detailed herein can be utilized in at least some exemplary embodiments.

In view of the above, it can be seen that in an exemplary embodiment, there is an implantable medical device, such as device 100, which can be a cochlear implant, a bone conduction implant, a middle ear implant, or any other type of implant, such as a pace maker or a device that needs recharging or telemetry, including a magnet apparatus, and a support body supporting the magnet apparatus (e.g., the body established by the silicone 199). In this embodiment, the magnet apparatus has a long axis and a short axis shorter than the long axis normal to the long axis. In an exemplary embodiment, at least one of the top surface or the bottom surface of the magnet apparatus (in FIGs. 20-22, both) establishes a curved outer periphery with respect to a cross-section lying on a plane on which the long axis lies, and which is parallel to the short axis (and can be lying on the short axis).

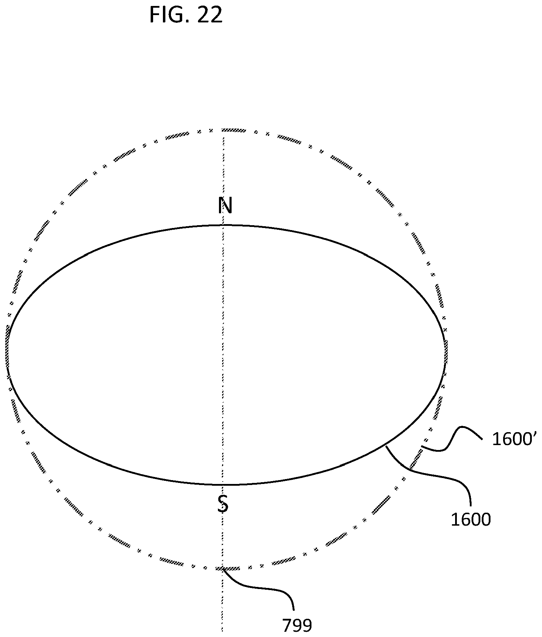

In the embodiment of FIGs 20-22, which depict a view from the side concomitant with that of FIG. 1C, the long axis runs horizontal (and, with respect to these embodiments, is the same throughout 360 degrees of orientation in the plane normal to the page), and the short axis runs vertical in the page. If we treat FIGs. 20-22 to also be cross-sections taken down the middle of the magnet apparatus (and the middle of implant 100), the short axis is maximum as shown, and the distance from the top to the bottom as measured parallel to the short axis decreases with distance from that center plane / with location outboard from the center. This also happens with the distance from the front to the back with distance from the long axis. It is noted that the side views presented in the figures before and including FIG. 22 are the same if the magnet is viewed from any of the four sides of the implant (as opposed to the top or the bottom). That said, in some embodiments, there can be arrangements where the magnet apparatus has two short axes. For example, when viewed from above, the magnet apparatus could look oval-shaped or elliptical shaped, instead of round. Further, when viewed from one side that is 90° from another side, the magnet apparatus could have more or less curvature or the like relative to the another side. Any shape that can enable the teachings herein can be used in some embodiments.

The shape of the magnet apparatus can be symmetrical about 1, 2, or 3 planes, where the planes can be normal to each other. The magnet apparatus can be rotationally symmetric about one axis, or can be rotationally symmetric about no axis, or can be rotationally symmetric about two axis, which can be normal to each other. In an embodiment, the magnet apparatus is rotationally symmetric about two axes but not about a third axis normal to the two axis, where all of these axes are normal to each other. In an embodiment, the magnet apparatus is rotationally symmetric about one axis but not about a second and a third axis all normal to each other.

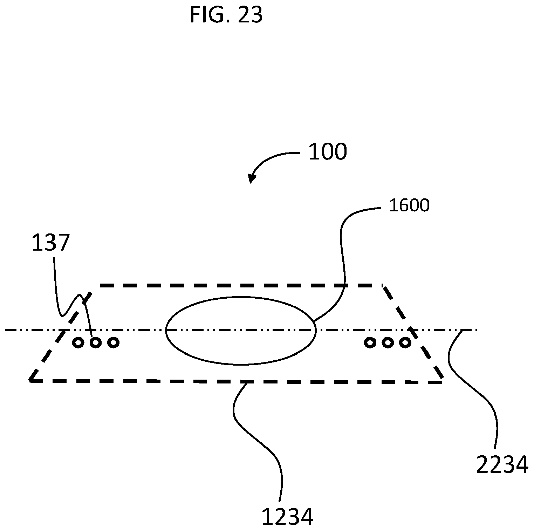

Consistent with the teachings above, in an exemplary embodiment, the magnet apparatus is magnetized in a direction of the short axis. In an exemplary embodiment, with respect to a first axis parallel to a base 1234 of the support body, the magnet apparatus is configured to rotate about the first axis parallel to the base. FIG. 23 depicts a front view of the implant 100 (the view when looking from the left in the perspective of FIG. 1C), depicting base 1234 (in an embodiment, the relative position of the axis can be a tangent surface of the base measured at a location beneath the magnet apparatus 1600, such as beneath the geometric center, etc.). Axis 2234 can be seen, which is parallel to the base 1234. Magnet apparatus 1600 can rotate about axis 2234. In an exemplary embodiment, the device is configured such that the magnet apparatus 1600 can rotate about a second axis 2238 normal to a first axis and parallel to the base.

In an exemplary embodiment, the device 100 is configured to effectively prevent rotation about an axis 2255 normal to the base and normal to the first axis. This can be done by creating channels within the elastomeric body that interface with, for example, cylindrical extension beams extending from sides of the magnet apparatus that permit rotation about two axes but not the third (the extension beam can travel in the channel to enable rotation, and the sides of the channel can prevent rotation in another direction).

In an exemplary embodiment, with respect to a plane perpendicular to a base of the support body, the device is configured to enable the magnet apparatus to rotate in the plane perpendicular to the base. In an exemplary embodiment, the device is configured to enable the magnet apparatus to rotate in a second plane normal to the plane perpendicular to the base. In an exemplary embodiment, the device is configured to effectively prevent rotation in a second plane normal to the plane perpendicular to the base / a second plane parallel to the base.

In an exemplary embodiment, the distance of the long axis is at least 5, 10, 15, 20, 25, 30, 35, 40, 45, 50, 55, 60, 65, 70, 75, 80, 90, 100, 125, 150, 175, 200, 250, 275, 300, 325, 350, 375, 400% or more or any value or range of values therebetween in 1% increments larger than the distance of the short axis (e.g., 28, 33, 38, 42, 41-57). In an exemplary embodiment, the distance of the long axis is no more than 5, 10, 15, 20, 25, 30, 35, 40, 45, 50, 55, 60, 65, 70, 75, or 80% or any value or range of values therebetween in 1% increments larger than the distance of the short axis. In an exemplary embodiment, the distance of the long axis is about 5, 10, 15, 20, 25, 30, 35, 40, 45, 50, 55, 60, 65, 70, 75, or 80% or any value or range of values therebetween in 1% increments larger than the distance of the short axis.

In an exemplary embodiment, the magnet apparatus has a maximum diameter of no more than or no less than or about equal to 5, 5.5, 6, 6.5, 7, 7.5, 8, 8.5, 9, 9.5, 10, 10.5, 11, 11.5, 12, 12.5, 13, 13.5, or 14 mm, or any value or range of values therebetween in 0.05 mm increments. Some embodiments might have a maximum diameter that is limited vis-à-vis an amount of rotation that would result so as to reduce the protrusion of the skin caused by, for example, full 90 degree rotation.

In an exemplary embodiment, the magnet apparatus has a minimum diameter of no less than or no more than or about equal to 2, 2.5, 3, 3.5, 4, 4.5, 5, 5.5, 6, 6.5, 7, 7.5 or 8 mm or any value or range of values therebetween in 0.1 mm increments. In an exemplary embodiment, this minimum diameter is measured normal to the plane of the base. The magnet can have two short axes of different lengths / distances, such as in the case where the magnet is not rotationally symmetric about the north-south pole. The short axes can have any of the minimum diameter values just detailed.

Various specific shapes of the magnet apparatus can be used. Indeed, in an exemplary embodiment, there is an implantable medical device, such as the cochlear implant 100, comprising a non-spherical magnet apparatus. This can be element 1600, or variations thereof (more on this below). The device has the aforementioned support body supporting the magnet apparatus. Here, the device is configured to enable the magnet apparatus to rotate relative to the support body when exposed to an external magnetic field such that a magnetic field of the magnet apparatus aligns more with the external magnetic field relative to that which would otherwise be the case. For example, initially, the initial misalignment could be 90 degrees, and the magnet apparatus can rotate so that that value is reduced by less than, equal to and/or greater than 10, 15, 20, 25, 30, 35, 40, 45, 50, 55, 60, 65, 70, 75, 80, 85, 90, 95, or 100% of the initial misalignment (if the change would be 45 degree misalignment, the value would be reduced by 50%).

In an exemplary embodiment, the implantable medical device is configured to limit the maximum rotation of the magnet apparatus relative to the zero rotation position with respect to one or both of the axes discussed above about which the magnet apparatus can rotate, depending on the embodiment. In an exemplary embodiment, the implant is configured such that a maximum amount of rotation that the magnet apparatus can experience from the normal position / zero degree rotation position, about one or two axes is 20.0, 21.0, 22.0, 23.0, 24.0, 25.0, 26.0, 27.0, 28.0, 29.0, 30.0, 31.0, 32.0, 33.0, 34.0, 35, 40, 45, 50, 55, 60, 65, 70, 71, 72, 73, 74, 75, 76, 77, 78, 79, 80, 81, 82, 83, 84, 85, 86, 87, 88, 89, or 90 degrees or any value or range of values therebetween in about 0.1 increments (e.g., about 49.3 to about 58.1 degrees, 67.3 degrees, etc.). This can be accomplished by, for example, utilizing tethers that attach to the outer boundaries of the magnet apparatus as well as to a plate or a beam or a beam apparatus that extends beneath the magnet apparatus within the elastomeric body a distance so that the plate establishes a sufficient anchoring for the tethers with respect to preventing further rotation of the magnet apparatus. The tethers could be located about the long axis spaced at 90 degree intervals. Alternatively, a more rigid system of sliding or telescoping beams could be used.

In any event, in an exemplary embodiment of this embodiment, the magnet apparatus is a modified sphere shape and/or the magnet apparatus is configured to rotate relative to the support body about more than one axis and/or about two axes normal to one another. Indeed, in an exemplary embodiment, because the magnetic field is not perfectly aligned in the length direction of the implant, the axis of rotation will be oblique and not normal to the length direction of the implant / will not be in a plane parallel to the length direction of the implant / will not be parallel to the long axis of the magnet.

As can be seen above, embodiments herein utilize unique shapes of the magnet apparatus - either the housing or the magnet or both. In an exemplary embodiment, as presented above, the magnet apparatus has a circular cross-section lying on a first plane normal to a north-south magnetization direction of the magnet apparatus and a non-circular and non-flat cross section lying on a second plane normal to the first plane. In an exemplary embodiment, the cross-section lying on the second plane can include a flat portion. In an exemplary embodiment, the cross-section lying on the second plane can include a circular portion. In an exemplary embodiment, the cross-section lying on the second plane can include a circular portion and a flat portion. In an exemplary embodiment, the cross-sections lying on the first plane can have any one or more the aforementioned features associated with the second plane. Corollary to this is that irrespective of the orientation of the implant in the head of the recipient, providing that the base is on the skull, the magnet can achieve alignment or partial alignment, via rotation, with the external magnetic field, in accordance with at least some of the embodiments herein, and the functionality associated therewith can be in this embodiment. There is also a north-south alignment magnet that is normal to the skin in normal operation (e.g., no torque), which can move / change orientation in Cartesian coordinates to change the direction of the alignment under the magnetic field. This embodiment can be analogous to how a top can move so that the axis points to different locations after a number of rotations / analogous to how the axis of the Earth changes over millennia (eventually, Polaris will not be the North Star, and then it will be such again, and so on). Not that the magnet rotates necessarily, but that the axis "wobble" or wanders in two planes.

In at least some exemplary embodiments, the magnet apparatus is a modified sphere shape. In an exemplary embodiment, the magnet itself and the housing or coating if present, also have a modified sphere shape. In an exemplary embodiment, speaking in general terms, conceptually or actually, the basis of the magnet apparatus is a sphere where portions on the top and/or the bottom of the sphere reduced / eliminated to arrive at the given modified sphere shape. Some embodiments, that is exactly how the magnet starts off, as a sphere, and then it is machined or otherwise worked on to obtain the modified sphere feature, and then it is clad with the aforementioned material to establish a housing or a coating etc., there about. That said, in an alternate embodiment, it is the design process that starts with the sphere and the designer works to achieve the desired modified sphere shape. That said, in an alternate embodiment, the design process starts with working towards the modified sphere shape as an initial matter. Still further, in an exemplary embodiment, the design could start with a modified cylinder shape as an initial matter. The corners could be rounded to obtain a utilitarian shape in a manner analogous to rounding a sphere to get the modified sphere.

In an exemplary embodiment, the modified sphere shape has a long diameter and a short diameter, as seen above. The long diameter of the modified sphere shape can be designed to be small enough that when the magnet rotates there is no pain for the recipient and/or a statistically significant number of recipients, such as the 10 to 90 percentile (or any range of values therebetween in one percentile increments) human factors male or female of natural born citizenship in the United States and/or the European Union as of January 1, 2019, who is older than 10, 11, 12, 13, 14, 15, 16, 17, 18, 19, 20, 21, 22, 23, 24, or 25 years of age and/or is younger than 90, 85, 80, 75, 70, 65, 60, or 55 years of age. In an exemplary embodiment, the pain factor can be established by a pain factor test that is permitted for use in the aforementioned jurisdictions on the aforementioned date and/or that is medically accepted as having utilitarian value for measuring pain. In an exemplary embodiment, to the extent there is pain or a sensation of movement of the magnet, the pain/sensation is no more than negligible or light or moderate according to the aforementioned pain factor tests.

In an exemplary embodiment, the short diameter of the magnet apparatus can have utilitarian value with respect to being small enough to fall within the desired implant thickness, which thickness can be less than equal to or greater than 2, 3, 4, 5, 6, 7, 8, 9, 10, 11, 12, 13, 14, 15, 16, 17, 18, 19, or 20 mm or any value or range of values therebetween in 0.1 mm increments. In an exemplary embodiment, there is utilitarian value if the overall volume of the magnet in the magnet apparatus is high enough to provide sufficient retention vis-à-vis magnet and the external component. In an exemplary embodiment, with respect to a separation distance of 1 cm from the surfaces of the magnet apparatus of the implant and the magnet apparatus of the external component, and attractive force will be at least 0.4, 0.5, 0.6. 0.7, 0.8, 0.9, 1.0, 1.1., 1.2, 1.3, 1.4, 1.5, 1.6, 1.7, 1.8, 1.9, 2, 2.25 or 2.5 Newtons or any value or range of values therebetween in 0.01 increments. In an exemplary embodiment, the magnet of the implant and/or the magnet of the external device is a magnet that produces a magnetic field of at least about 0.01, 0.015, 0.02, 0.025, 0.03, 0.035, 0.04, 0.045, 0.05,0.055, 0.06, 0.07, 0.08, 0.09, 0.1, 0.15, 0.2, 0.25, 0.3, 0.35, or 0.4 or any values or range of values therebetween in 0.001 T increments. In an exemplary embodiment, the external magnet produces a magnetic field of at least about 0.05, 0.1, 0.15, 0.2, 0.25, 0.3 T or more or any value or range of values therebetween in 0.001T increments.

In an exemplary embodiment, the magnet of the implant and/or the external magnet has a volume of less than, greater than or about equal to150, 175, 200, 225, 250, 275, 300, 325, 350, 375, 400, 425, 450, 475 or 500 mm³ or any value or range of values therebetween in 1 mm³ increments.

Embodiments include exposing the implant and thus the magnet therein to at least a 1, 1.5, 2, 2.5, 3, 3.5, 4, 4.5, 5, 5.5 or 6T or any value or range of values therebetween in 0.1 T increments magnetic field of an MRI machine for at least 5, 10, 15, 20, 25, 30, 35, 40, 45, 50, 55, 60, 70, 80, or 90 or more minutes, or any value or range of values therebetween in 1 minute increments without any external holding device, such as a splint or a bandage. In an exemplary embodiment, the magnet does not rotate about an axis normal to the bottom of the implant or rotates no more than 1, 2, 3, 4, 5, 6, 7, 8, 9, or 10 degrees, or any value or range of values therebetween in 0.1 degree increments even though the initial misalignment of the magnetic field with the poles of the magnet is 1, 1.5, 2, 2.5, 3, 3.5, 4, 4.5, 5, 5.5, 6, 6.5, or 7 times that amount and/or any of the angles detailed herein.

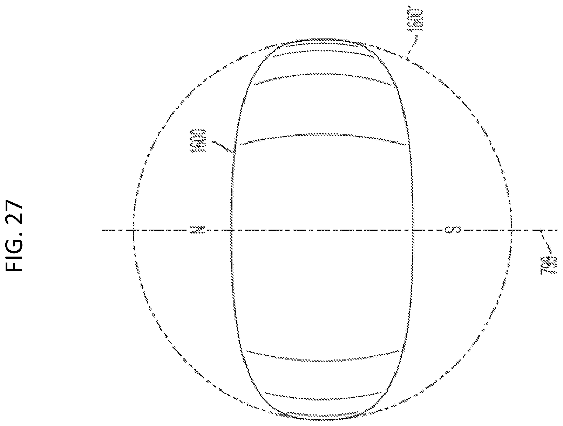

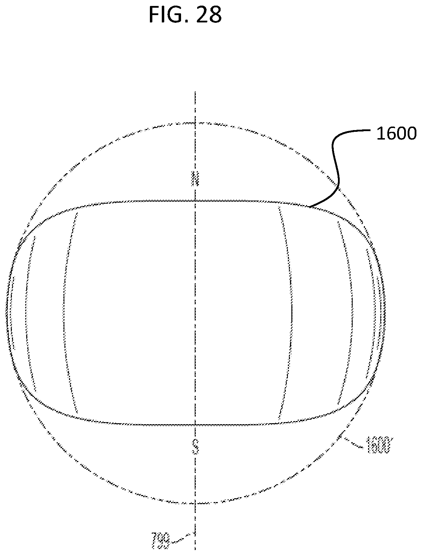

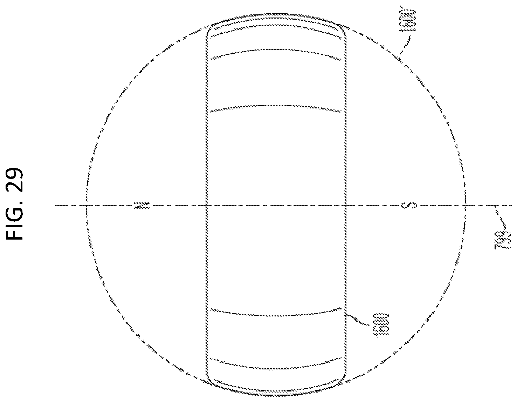

Embodiments of the magnet apparatus can have various shapes and/or various sizes. Figures 27 and 28 present some additional exemplary embodiments of the magnet apparatus 1600 relative to a perfect sphere 1600'. In this exemplary embodiment, it can be seen that a portion of the magnet apparatus has a spherical shape and a portion of the magnet apparatus has an elliptical shaped. This as contrasted to the embodiment of figure 20, which is a purely elliptical shape. FIG. 29 presents an exemplary embodiment where the magnet apparatus is part spherical and most of the rest is planar (curvature is present to avoid the sharp edge at the end of the plane - a chamfer or the like can be used in some other embodiments).

In an exemplary embodiment, the outer surfaces of the magnet apparatus is faceted instead of curved, in part or in full. Any shape disclosed herein in full or in part can instead be a shape where some portion or all portions of the curve(s) are instead facets of flat surfaces (curved edges can be used to "connect" the surfaces).

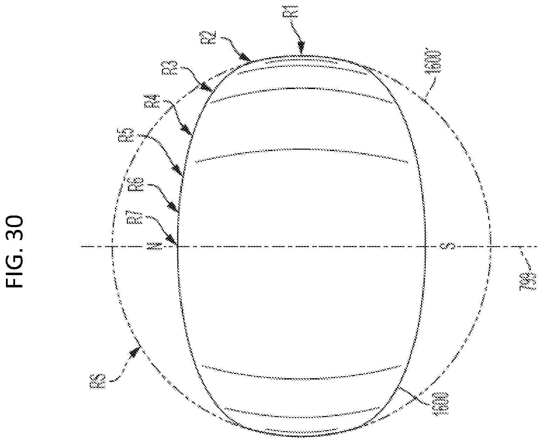

FIG. 30 presents a diagram for explanatory purposes depicting how an exemplary magnet apparatus 1600 can have compound outer surfaces. In this regard, it can be seen that the magnet apparatus can have an ever decreasing radius of curvature. In an exemplary embodiment R1 is the radius of the sphere 1600 prime, R2, R3, R4, R5, R6 and/or R7 is tighter / smaller than R1, such as being for example, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99%, or any value or range of values therebetween in 0.1% increments of the value of R1. In an exemplary embodiment R3, R4, R5, R6 and/or R7 is tighter / smaller than R2, such as being for example, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99% or any value or range of values therebetween in 0.1% increments of the value of R2. In an exemplary embodiment R4, R5, R6, and/or R7 is tighter / smaller than R3, such as being for example, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99% or any value or range of values therebetween in 0.1% increments of the value of R3. In an exemplary embodiment, R5, R6 and/or R7 is tighter / smaller than R4, such as being for example, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98 or 99% or any value or range of values therebetween in 0.1% increments of the value of R4, and so on. That said, as can be seen in the figures, R5, R6 and R7 can have progressively increasing values relative to R4. In an exemplary embodiment R5, R6, and/or R7 is looser / larger than R4, such as being for example, 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99%, or any value or range of values therebetween in 0.1% increments of the value of R4, and so on. Any value of R1, R2, R3, R4, R5, R6, and R7 can be larger or smaller than the preceding value or the proceeding value by 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99%, or any value or range of values therebetween in 0.1% increments.

In some instances, the above is not the case. R1 can be smaller than R2 or R3 or R4 or R5 or R6 or R7, and so on, such as by any of the percentages herein relative to any of the other radii.

In an exemplary embodiment, a value of the surface area that is greater than less than or equal to 100, 99, 98, 97, 96, 95, 94, 93, 92, 91, 90, 85, 80, 75, 70, 65, 60, 55, 50, 45, 40, 35, 30, 25, 20, 19, 18, 17, 16, 15, 14, 13 12, 11, or 10 percent of the total surface area of the magnet apparatus is curved. In an exemplary embodiment, a cross-section taken normal to the bottom surface of the implant through a center of the magnet apparatus has an outer profile where an amount that is greater than less than or equal to 100, 99, 98, 97, 96, 95, 94, 93, 92, 91, 90, 85, 80, 75, 70, 65, 60, 55, 50, 45, 40, 35, 30, 25, 20, 19, 18, 17, 16, 15, 14, 13 12, 11, or 10 percent of the total outside of the cross-section of the magnet apparatus is curved. In an exemplary embodiment, the cross-section is non-rectangular and/or non-trapezoidal.

In an exemplary embodiment, R1 and/or R2 and/or R3 can have a radius of curvature that is larger than the radius of the sphere RS, where the local diameter of the magnet apparatus can be the same as the diameter of the sphere 1600'. In an exemplary embodiment, R1, R2, and/or R3 is 1, 2, 3, 4, 5, 6, 7, 8, 9, 10, 15, 20, 25, 30, 35, 40, 45, 50, 55, 60, 65, 70, 75, 80, 85, 90, 91, 92, 93, 94, 95, 96, 97, 98, or 99%, or any value or range of values therebetween in 0.1% increments larger than the local radius of curvature of the sphere 1600'.

Any shape that can enable the teachings detailed herein can be utilized in at least some exemplary embodiments.

It is noted that the arrow heads of figure 30 are arrayed along the vectors that constitute 15° increments between the 12 o'clock and the 3 o'clock position as measured from the X axis. R1 would be at angle 0°, R2 would be at angle 15°, R3 would be at angle 30° and so on.

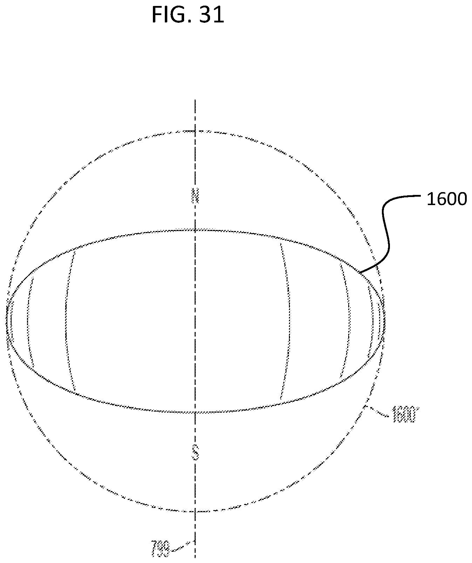

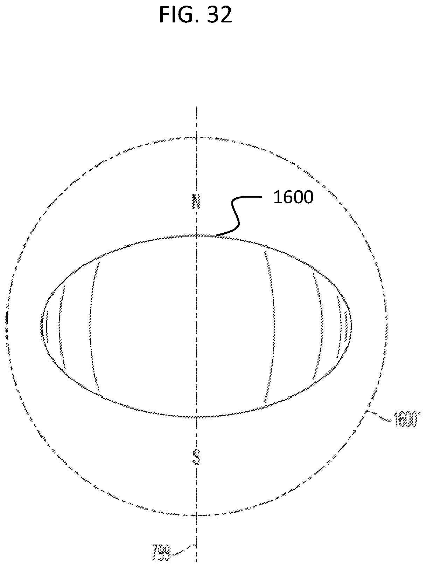

Again, while some embodiments are rotationally symmetric about axis 799, in other embodiments, this is not the case. FIG. 31 presents an exemplary embodiment which is the side view of the embodiment of figure 27. As can be seen, the side of the embodiment of figure 31 27 is more elliptical than that of the side shown in figure 27. FIG. 32 presents another exemplary embodiment which is the side view of the embodiment of figure 27. As can be seen, this has a second small axis that is larger than the small axis seen in the side view of figure 27. The values associated with figure 30 detailed above can be applicable to any side and, accordingly, a given side can have different values with respect to another side.

Any of the values detailed herein can be applicable to any embodiment disclosed herein providing that the art enables such. Thus, the above noted major axis dimensions can correspond to the diameter of the sphere 1600' of FIGs. 27-31 and thus the maximum diameter of the magnet apparatus 1600. Thus, the small axis dimensions detailed herein can correspond to the height of the magnet apparatus shown in the figures.

In an exemplary embodiment, the medical device is configured such that a 3 T magnetic field exerting a force on the magnet apparatus moves the long axis of the magnet apparatus relatively perpendicular to the external magnetic field when the device is implanted between bone and the surface of the skin. In an exemplary embodiment, the medical device is configured such that in a relaxed position (e.g., zero rotation), a long axis of the magnet apparatus is relatively parallel to a surface of the skin immediately above the magnet apparatus when the device is implanted between bone and the surface of the skin. The device can also be configured such that the long axis of the magnet apparatus is relatively parallel to a tangent plane at the surface of the skin immediately above the magnet apparatus when the device is implanted between bone and the surface of the skin. The relaxed position can be present in the absence of an external magnetic field, such as an MRI field. In an exemplary embodiment, the medical device is configured such that a 1, 1.5, 2, 2.5, or 3 T magnetic field aligned parallel to the surface of skin immediately above the magnet apparatus moves the long axis of the magnet apparatus relatively perpendicular to the surface of the skin when the device is implanted between bone and the surface of the skin. The movement can be any of the rotations detailed herein in some embodiments. This is not to say that the magnet will not move / rotate if the field is aligned differently. Indeed, in an exemplary embodiment, in addition to the above proviso, the medical device is configured such that a 1, 1.5, 2, 2.5, or 3 T magnetic field aligned parallel to the surface of skin immediately above the magnet apparatus moves the long axis of the magnet apparatus obliquely to the surface of the skin when the device is implanted between bone and the surface of the skin, wherein the oblique angle can be any value or range of values between zero and 70 degrees (not inclusive) in 1 degree increments. The movement can be any of the rotations detailed herein in some embodiments. Alternatively, in an exemplary embodiment, the medical device is configured such that a 1, 1.5, 2, 2.5, or 3 T magnetic field aligned perpendicular to the surface of skin immediately above the magnet apparatus does not move the long axis of the magnet apparatus relative to the surface of the skin when the device is implanted between bone and the surface of the skin, and/or the movement is less than 30, 25, 20, 15, 10, 9, 8, 7, 6, 5, 4, 3, 2, or 1 degrees.

In an exemplary embodiment, the implantable medical device is configured such that upon the elimination of a 3 T (or any of the above aforementioned fields, in some embodiments) magnetic field, the magnet apparatus moves the long axis of the magnet apparatus back towards the relatively parallel to the surface of the skin orientation when the device is implanted between bone and the surface of the skin. In an exemplary embodiment, it moves it to within 10, 9, 8, 7, 6, 5, 4, 3, 2, 1, or zero degrees of the previous orientation, or any value or range of values therebetween in 0.1 degree increments. Also, in an exemplary embodiment, the body includes elastic features that hold the magnet apparatus with the long axis relatively parallel to the surface of the skin in the absence of the 3 T magnetic field and returns the long axis to the relatively parallel orientation upon the elimination of the 3 T magnetic field and/or holds and returns the long axis to within 10, 9, 8, 7, 6, 5, 4, 3, 2, 1, or zero degrees or any value or range of values therebetween in 0.1 degree increments of the parallel orientation.

It is noted that any disclosure herein relating to orientation to the skin corresponds to an alternate embodiment relating to orientation of the base, as, in some embodiments, the base is parallel to skin of the recipient in a local manner, in a manner analogous to the outside of the skin being parallel to the skull bone beneath the skin in locations behind the pinna / above the mastoid bone of a person.

In an exemplary embodiment, the device is configured to enable the magnet apparatus to tumble within the support body.

An exemplary embodiment includes an implantable medical device, comprising a support body and a magnet apparatus, wherein the support body includes a portion made of an elastomeric material (e.g., silicone) that at least partially envelops the magnet apparatus and elastically deforms to enable the magnet apparatus to rotate about an axis parallel to the base of the device / rotate in a plane normal to the base at least 40, 45, 50, 55, 60, 65, 70, 75, 80, 85, or 90 degrees or more or any value or range of values therebetween in 1 degree increments from a relaxed orientation when subjected to a magnetic field of at least 1, 1.5, 2, 2.5, or 3 T that is oriented normal to a north-south magnetic axis of the magnet apparatus and normal to the axis / parallel to the plane. Again, this is not to say that the magnet requires the magnetic field to be aligned as just detailed. This is to say that if the magnetic field is aligned as just detailed, the magnet will rotate accordingly. The magnet can rotate with respect to the magnetic fields that are aligned in different manners, such as detailed above. In some embodiments, the portion made of an elastomeric material elastically deforms to enable the magnet apparatus to rotate about the axis parallel to a base of the device no more than 90 degrees from the relaxed orientation when subjected to a magnetic field of at least 3 T that is oriented normal to the north-south magnetic axis of the magnet apparatus and normal to the axis.

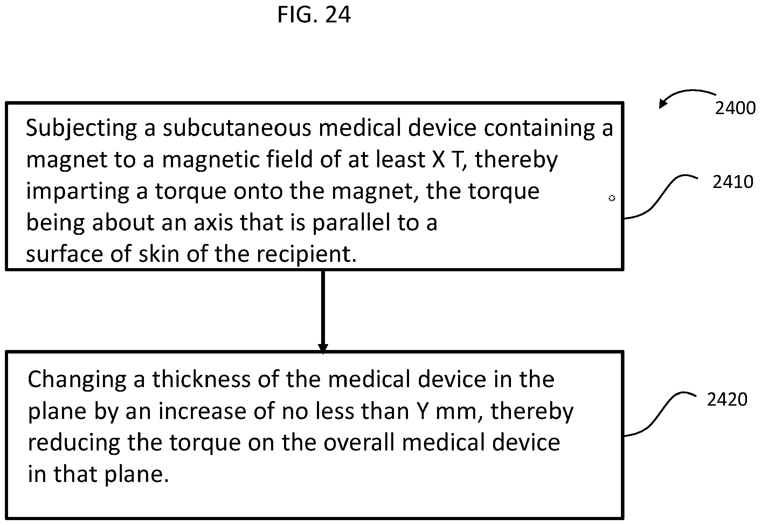

FIG. 24 presents an exemplary flowchart for an exemplary non-claimed method, method 2400, which includes method action 2410, which includes subjecting a subcutaneous medical device containing a magnet to a magnetic field of at least X T, thereby imparting a torque onto the magnet, the torque being about an axis (e.g., axis 2238 and/or axis 2234) that is parallel to surface of skin of the recipient. In an embodiment, X equals 0.5, 1, 1.5, 2, 2.5, 3, 3.5, 4, 4.5, or 5 or more or any value or range of values therebetween in 0.1 increments. Method 2400 also includes method action 2420, which includes changing a thickness (or, allowing a thickness to change) of the medical device in a direction normal to the axis by an increase of no less than Y mm, thereby reducing the torque on the overall medical device in that plane, where Y equals 1, 1.25, 1.5, 1.75, 2, 2.25, 2.5, 2.75, 3, 3.25, 3.5, 3.75, 4, 4.5, 5, 5.5, 6, 6.5, 7, 7.5, 8, 8.5, or 9, or any value or range of values therebetween in 0.01 mm increments. In an exemplary embodiment, the action of changing the thickness of the medical device results from the magnet rotating about the axis (e.g., one of axis 2238 and axis 2234) while also being able to rotate about a second axis normal to the axis (e.g., the other of axis 2238 and 2234).

The change in thickness can vary depending on the amount of rotation of the magnet.

In an exemplary embodiment, the action of changing the thickness of the medical device in the plane by an increase of no less than Y mm includes doing so by an increase of no more than Y plus 0.25, 0.5, 0.75, 1, 1.25, 1.5, 1.75, 2, 2.25, 2.5, 2.75, 3, 3.25, 3.5, 3.75, 4, 4.5, 5, 5.5, 6, 6.5, 7, 7.5, or 8 or any value or range of values therebetween in 0.01 mm increments.

In an exemplary embodiment, the reduction in torque is reduced by at least and/or an amount equal to 10, 15, 20, 25, 30, 35, 40, 45, 50, 55, 60, 65, 70, 75, 80, 81, 82, 83, 84, 85, 86, 87, 88, 89, 90, 91, 92, 93, 94, 95, 96, 97, 98, 99, or 100%, or any value or range of values therebetween in 0.1% increments relative to that which would be the case in the absence of the of the change in thickens and/or relative to that which would be the case if the magnet was held stationary. Thus, in an exemplary embodiment, the action of changing the thickness of the medical device results in the reduction of but not the elimination of the torque on the overall medical device about the axis by permitting the magnet to rotate about the axis. Conversely, in an alternate embodiment, there is total elimination of the torque on the overall medical device.

In an exemplary embodiment, the initial torque and/or the torque that would exist in the absence of the thickness change is 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.9, 1.0, 1.25, 1.5 or more newton-meters or any value or range of values therebetween in 0.01 newton-meter increments.

In an exemplary embodiment, changing the thickness of the medical device by the above noted values includes doing so over an area that is no more than Z mm in a shortest direction of the area, where Z can be 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or 15, or any value or range of values therebetween in 0.1 increments. It is noted that in some embodiments, the area can have a larger direction of area than the shortest direction of area, and thus can exceed these values. In an alternate embodiment, the above noted values can be for the largest direction of the area. The different directions of area can be because the magnet apparatus can have different geometries in different axes.

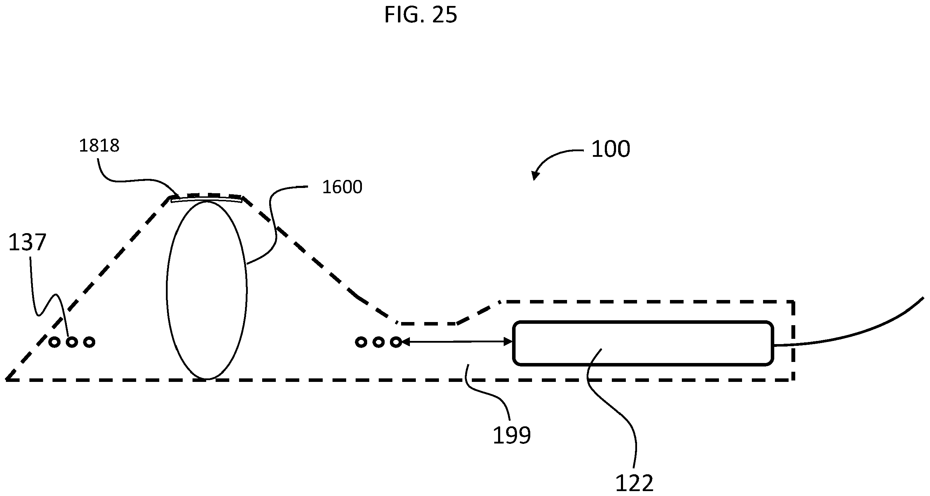

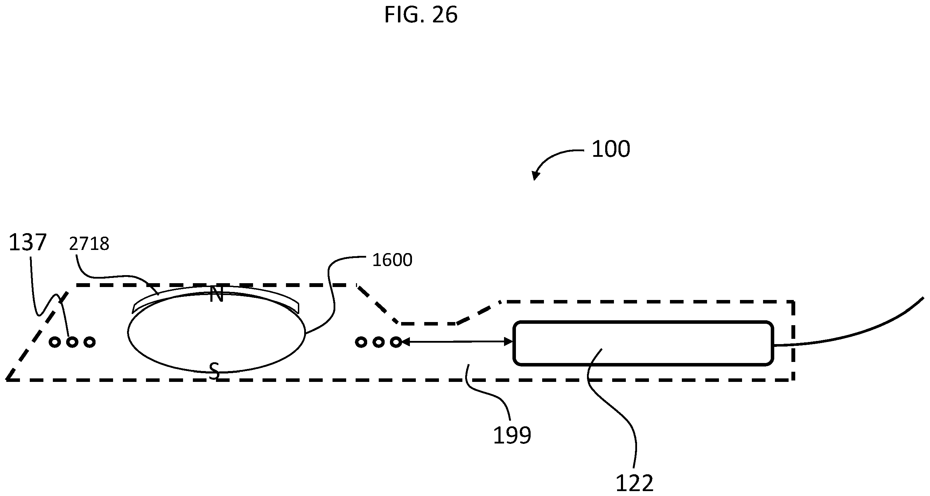

FIG. 25 depicts an alternate exemplary embodiment of the implant 100, which includes a plate 1818 embedded in the elastomeric body. Thus, an exemplary embodiment includes a device where there is a plate located inside the support body between the magnet apparatus and the surface of the skin. In this exemplary embodiment, the plate diffuses force / spreads out force within the body upon rotation of the magnet apparatus relative to that which would otherwise be the case. This concept can be understood easily from FIG. 25, which shows the plate having a greater surface area at the top relative to the surface area at issue with respect to the magnet apparatus 1600. Thus, the stress applied to the local area of the silicone / elastomeric material is lower relative to that which would otherwise be the case if the plate was not present and instead the magnet apparatus 1600 directly interfaced with the elastomeric material at at least some of the locations where the plate so interfaces. In an exemplary embodiment, the stress applied to the elastomeric material at at least some locations proximate the plate 1818 is reduced by less than, greater than or about equal to 5, 10, 15, 20, 25, 30, 35, 40, 45, 50, 55, 60, 65, 70, 75, 80, 85, 86, 87, 88, 89, 90, 91, 92, 93, 94, or 96%, or any value or range of values therebetween in 1% increments. This stress reduction can also reduce the pressure that is applied to the skin of the recipient by any of these amounts as well relative to that which would otherwise be the case in the absence of the plate 1818. FIG. 26 presents an alternate embodiment of a plate, plate 2718, which can aid in holding the magnet still during normal operation (e.g., holding the external device to the recipient / removal / attachment of the external device, etc.) but still permit the magnet apparatus to rotate as detailed herein.

The plate can be made of plastic or titanium or any material that can enable application.

In an exemplary embodiment, the plate 1818 is static relative to the local portions of the elastomeric material that interface there with. In an exemplary embodiment, the plate can have debits or through holes of the like for the elastomeric material to extend through thus securing the plate within the elastomeric body so that the plate will not move relative to the local portions of the last body. In an exemplary embodiment, the plate has a maximum diameter and/or has diameters that are normal to each other that are less than, greater than or about equal to 5, 10, 15, 20, 25, 30, 35, 40, 45, 50, 55, 60, 65, 70, 75, 80, 85, 86, 87, 88, 89, 90, 91, 92, 93, 94, 95, 96, 97, 98, 99, 100, 101, 102, 103, 104, 105, 106, 107, 108, 109, 110, 115, 120, 125, 130, or 135%, or any value or range of values therebetween in 1% increments of the large diameter of the magnet apparatus 1600. In an exemplary embodiment, the plate 1818 is a flat plate, while in other embodiments, the plate 1818 is curved on one or both sides. In an exemplary embodiment, the plate can have a concave surface relative to the magnet apparatus 1601 the side facing the magnet apparatus and/or can have a convex surface relative to the magnet apparatus one the side facing away from the magnet apparatus. Any arrangement that can have utilitarian value can be utilized in some embodiments.

In an exemplary embodiment, the plate alone or in combination with other structure, such as the tethers or the like, can be a component that prevents or otherwise limits the maximum rotation of the magnet apparatus to any of the values detailed above or any other value that might have utilitarian value. In an exemplary embodiment, the plate can include protrusions that could interface with tracks or channels and the magnet apparatus to guide the magnet apparatus relative to the plate and/or to act as stops for rotation such as that which may occur when the protrusion reaches an end of the channel.

In an exemplary embodiment, there is an implantable medical device, comprising a magnet apparatus having a long axis and a short axis and a support body supporting the magnet apparatus, wherein the device is configured to enable the magnet apparatus to rotate relative to the support body in a plane on which the long axis and the short axis lie when exposed to an external magnetic field such that a magnetic field of the magnet apparatus aligns more with the external magnetic field relative to that which would otherwise be the case.

In some embodiments, there is only one plate located at the top of the implantable component, as shown in figure 25. In other embodiments, there are two plates, one located at the top of one located at the bottom. Indeed, in an exemplary embodiment, a bottom plate can defuse the force that is created during rotation in a manner analogous to the plate 1818. In an exemplary embodiment, this can provide diffusion of the force over a larger area of the bone as well. Any of the above noted features associated with the top plate can be applicable to the bottom plate. In an exemplary embodiment, the bottom plate can be much larger than the top plate, such as at least 1.5, 2, 2.5, 3, 3.5, 4, 4.5, 5, 5.5, 6 or more times the size of the top plate with respect to one or more diameters.

The embodiments depicted in the figures and described above have utilized the silicone body / elastomeric body to directly support the magnet apparatus 1600. The body can extend about part of the circumference or all of the circumference of the magnet apparatus. In an exemplary embodiment, a chassis can be used to support the magnet apparatus. In an embodiment, this chassis can be a separate component from the body which can be a single component or two separate components or more than two separate components spaced apart from one another that can be or may not be held directly to each other by a structure other than the silicone body. In an embodiment, this chassis or the like can be embedded within at least partially embedded within the elastomeric body, which chassis can in turn support the magnet apparatus 1600 and otherwise allow the magnet apparatus to rotate. In this regard, booms or the like or telescopic tubes can be utilized to hold the magnet apparatus to the chassis but also permit the magnet apparatus to rotate. In an exemplary embodiment, instead of a chassis that supports directly the magnet apparatus, there is more of an indirect support structure that extends about at least a portion of the circumference of the magnet apparatus. In an exemplary embodiment, the support structure can be two or more components that are or are not connected directly to one another beyond that which results from the silicone body, concomitant with the chassis detailed above.

In an exemplary embodiment, the structural components / chassis or another component can utilize a magnetic field to "hold" or "guide" the magnet apparatus 1600. In this regard, in an exemplary embodiment, additional magnets and/or additional magnetic components (the components need not be magnetic - they could be components that are simply attracted to the magnet - the attraction, combined with proper placement of these components within the silicone body can provide a structure that results in at least a guiding force being applied to the magnet apparatus of the like. In this regard, these components can be placed in various areas beyond those which would be governed by alignment with the poles of the magnet apparatus which could result if the components were also magnets. That said, in an exemplary embodiment, magnet apparatus is with poles that are not aligned can be utilized to impart a holding force or a guiding force onto the magnet apparatus 1600.

In at least some exemplary embodiments, the implanted magnet will never be demagnetized by strong MRI magnetic field because it is free to rotate to align with the field in accordance with the teachings detailed herein. By aligning the polarity of the magnet normal to the skin surface, a stronger magnetic field can be obtained relative to that which would otherwise be the case if the poles were aligned diametrically. For magnets having the same volume and/or the same maximum diameter, made of the same material and/or of the same magnetization imparting technique and/or magnetized to a maximum, the magnet having the alignment in accordance with figure 9 will have at least a 1.25, 1.5, 1.75, 2, 2.25, 2.5, 2.75, 3, 3.25, 3.5, 3.75, 4, 4.25, 4.5, 4.75, or 5 times as strong magnetic force attraction to an external component relative to a diametrically opposed alignment of that design (or, for example, FIG. 5 vs. FIG. 6A), all other things being equal.

In an exemplary embodiment, the implantable component can be MRI compatible for a magnetic field according to at least one or more of the magnetic field strengths detailed herein in accordance with the FDA regulations of the United States of America and/or the comparable regulations in any one or more of the states thereof and/or one or more of the European Union countries. In an exemplary embodiment, the modified sphere shapes according to the teachings detailed herein and/or the other shapes can be more volumetrically efficient than a disk magnet. Thus, the overall surface area of the housing / shell containing the magnet can be lower for the same volume of magnetic material relative to a disk-shaped magnet, such as at least 5, 10, 15, 20, 25, 30, 35, 40, 45, or 50%, or more or any value or range of values therebetween in 1% increments.

Exemplary embodiments can include a non-claimed method of utilizing an external component that has been used and/or of a design that has been used with an implantable component having a magnet in the form of a disk magnet having axially aligned polarity with the magnets detailed herein. In this regard, for example, the teachings detailed herein can be used to design upgrade / design retrofit existing implants (as opposed to modifying an existing implantable component - more on this in a moment). In this regard, in some embodiments, there are existing designs of implants where everything is the same except the magnet that is used and the accompanying features, where the magnet is according to the teachings herein. It is noted that in some embodiments, there is prevention of rotation of the magnet about an axis normal to the base of the implant. That said, in some other embodiments, there are methods of retrofitting actually existing implantable components. This can include taking an existing magnet and removing such and then replacing that magnet with the teachings detailed herein, and, if utilitarian, making modifications, so that the implant will support the new magnet.

In an exemplary embodiment, for a desired given magnetic retention force and for a given implanted coil, the distance from the magnet to the implanted coil is at least 5, 10, 15, 20, 25, 30, 35, 40, 45, or 50%, or more or any value or range of values therebetween in 1% increments relative to that which would be the case for a disk magnet (e.g., of a retrofitted (design or existing) implant), where the disk magnet has a thickness of 1, 1.5, 2., 2.5, 3, 3.5, or 4 mm, or any value or range of values therebetween in 0.1 mm increments. Other than the shape, the amount of magnetic material, the magnetization, etc. can be the same, for comparison. That is, the comparison is an "all other things being equal comparison" in some embodiments. For a desired given retention force and for a given implant coil, the distance from the magnet to the implanted coil is at least 5, 10, 15, 20, 25, 30, 35, 40, 45, or 50% or more larger or any value or range of values therebetween in 1%, relative to that which would be the case for a disk magnet having a thickness as just detailed, all other things being equal.

It is noted that any method detailed herein also corresponds to a disclosure of a device and/or system configured to execute one or more or all of the method actions detailed herein. It is further noted that any disclosure of a device and/or system detailed herein corresponds to a method of making and/or using that the device and/or system, including a method of using that device according to the functionality detailed herein. Embodiments can exclude a cylindrical, plate, disk and spherical magnet, in the implant and/or in the external device but can also include a device that has such in the external device. Embodiments can exclude diametrically aligned magnetic pole magnets in the implant and/or in the external device.

It is further noted that any disclosure of a device and/or system detailed herein also corresponds to a disclosure of otherwise providing that device and/or system.

It is noted that in at least some exemplary embodiments, any feature disclosed herein can be utilized in combination with any other feature disclosed herein unless otherwise specified. Accordingly, exemplary embodiments include a medical device including one or more or all of the teachings detailed herein, in any combination.

Note that exemplary embodiments include components detailed herein and in the figures that are rotationally symmetric about an axis thereof. Accordingly, any disclosure herein corresponds to a disclosure in an alternate embodiment of a rotationally symmetric component about an axis thereof. Moreover, the exemplary embodiments include components detailed in the figures that have cross-sections that are constant in and out of the plane of the figure. Thus, the magnet apparatus 160 can correspond to a bar or box magnet apparatus, etc.).

Any disclosure herein of any component and/or feature can be combined with any one or more of any other component and/or feature disclosure herein unless otherwise noted, providing that the art enables such. Any disclosure herein of any component and/or feature can be explicitly excluded from combination with any one or more or any other component and/or feature disclosed herein unless otherwise noted, providing that the art enables such. Any disclosure herein of any method action includes a disclosure of a device and/or system configured to implement that method action. Any disclosure herein of a device and/or system corresponds to a disclosure of a method of utilizing that device and/or system. Any disclosure herein of a manufacturing method corresponds to a disclosure of a device and/or system that results from the manufacturing method. Any disclosure of a device and/or system corresponds to a disclosure of a method of making a device and/or system.

Any disclosure herein could be further modified to include components enabling removal of the magnet - if for example an MRI of a part of the body in the vicinity of the implant is required and the magnet creates an artifact. This could be achieved for example by having the feature contained in a module which is reversibly separable from the rest of the implant 100 or having an opening (or means of creating and opening) somewhere in the elastomer through which the magnet could be removed.

Any disclosure herein could be further modified to have the polarity of magnetization at an oblique non-90 degree angle form the short axis.

While various embodiments of the present invention have been described above, it should be understood that they have been presented by way of example only, and not limitation. It will be apparent to persons skilled in the relevant art that various changes in form and detail can be made therein without departing from the scope of the invention.

## Claims

1. An implantable medical device (100), comprising:
a magnet apparatus (1600); and
a support body supporting the magnet apparatus, wherein
the magnet apparatus has a long axis and a short axis shorter than the long axis normal to the long axis, and
at least one of the top surface or the bottom surface of the magnet apparatus establishes a curved outer periphery with respect to a cross-section lying on a plane on which the long axis lies and which is parallel to the short axis,
**characterized in that**
the support body includes a monolithic portion made of elastomeric material (199) that at least partially envelops the magnet apparatus and positions the magnet apparatus such that the magnet apparatus is biased in a direction such that the long axis is generally parallel to a base of the device, or
the support body includes a monolithic portion made of elastomeric material (199) that at least partially envelops the magnet apparatus and elastically deforms to enable the magnet apparatus to rotate in a plane normal to a base of the device at least 45, 60, 70, 85 or 90 degrees from a relaxed orientation when subjected to a magnetic field of at least 1 T.

2. The implantable medical device of claim 1, wherein:
the magnet apparatus is magnetized in a direction of the short axis.

3. The implantable medical device of claim 1, wherein:
with respect to a first axis parallel to a base of the support body, the device is configured such that the magnet apparatus can rotate about the first axis parallel to the base.

4. The implantable medical device of claim 3, wherein:
the device is configured such that the magnet apparatus can rotate about a second axis normal to a first axis and parallel to the base, or
the device is configured to effectively prevent rotation about a second axis normal to the base and normal to the first axis.

5. The implantable medical device of claim 1, wherein:
the distance of the long axis is at least 33% larger than the distance of the short axis.

6. The implantable medical device of claim 1, wherein:
the implantable medical device is a receiver stimulator of a cochlear implant; and the magnet apparatus has a maximum diameter of no more than 8 mm and/or a minimum diameter of no less than 3 mm.

7. The implantable medical device according to claim 1, wherein:
the magnet apparatus is a non-spherical magnet apparatus;
the device is configured to enable the magnet apparatus to rotate relative to the support body when exposed to an external magnetic field such that a magnetic field of the magnet apparatus aligns more with the external magnetic field relative to that which would otherwise be the case, and
at least one of:
the magnet apparatus is a modified sphere shape; or
the magnet apparatus is configured to rotate relative to the support body about more than one axis.

8. The implantable medical device of claim 7, wherein:
the medical device is configured such that in a relaxed position, a long axis of the magnet apparatus is relatively parallel to a surface of the skin immediately above the magnet apparatus when the device is implanted between bone and the surface of the skin.

9. The implantable medical device of claim 8, wherein:
the medical device is configured such that a 3 T magnetic field aligned parallel to the surface of skin immediately above the magnet apparatus moves the long axis of the magnet apparatus relatively perpendicular to the surface of the skin when the device is implanted between bone and the surface of the skin, and
the medical device is configured such that upon the elimination of the 3 T magnetic field the magnet apparatus moves the long axis of the magnet apparatus back towards the relatively parallel to the surface of the skin orientation when the device is implanted between bone and the surface of the skin.

10. The implantable medical device of claim 7, wherein:
the body includes elastic features that hold the magnet apparatus with a long axis relatively parallel to the surface of the skin in the absence of the 3 T magnetic field and returns the long axis to the relatively parallel orientation upon the elimination of the 3 T magnetic field.

11. The implantable medical device, of claim 7, wherein:
the device is configured to enable the magnet apparatus to tumble within the support body.

12. The implantable medical device of claim 8, wherein:
a plate is located inside the support body between the magnet apparatus and the surface of the skin, which plate diffuses force throughout the body upon rotation of the magnet apparatus relative to that which would otherwise be the case, and/or
the magnet apparatus has a circular cross-section lying on a first plane normal to a north-south magnetization direction of the magnet apparatus and a non-circular and non-flat cross section lying on a second plane normal to the first plane.

13. The implantable medical device, of any one of the claims 1-12, wherein:
the device is configured to avoid a top-dead-center position of the magnet apparatus.

## Patentansprüche

1. Implantierbare medizinische Vorrichtung (100), die Folgendes umfasst:
eine Magnetvorrichtung (1600); und
einen Trägerkörper, der die Magnetvorrichtung trägt, wobei
die Magnetvorrichtung eine lange Achse und eine kurze Achse hat, die kürzer als die lange Achse und senkrecht zur langen Achse ist, und
die obere Oberfläche und/oder die unteren Oberfläche der Magnetvorrichtung einen gekrümmten Außenumfang in Bezug auf einen Querschnitt aufweist, der auf einer Ebene liegt, auf der die lange Achse liegt und die parallel zu der kurzen Achse ist,
**dadurch gekennzeichnet, dass**
der Trägerkörper einen monolithischen Abschnitt aus elastomerem Material (199) enthält, der die Magnetvorrichtung zumindest teilweise umhüllt und die Magnetvorrichtung so positioniert, dass die Magnetvorrichtung in eine Richtung vorgespannt ist, so dass die lange Achse im Allgemeinen parallel zu einer Basis der Vorrichtung ist, oder
der Trägerkörper einen monolithischen Abschnitt aus elastomerem Material (199) enthält, der die Magnetvorrichtung zumindest teilweise umhüllt und sich elastisch verformt, damit sich die Magnetvorrichtung in einer Ebene senkrecht zu einer Basis der Vorrichtung um mindestens 45, 60, 70, 85 oder 90 Grad aus einer entspannten Ausrichtung drehen kann, wenn sie einem Magnetfeld von mindestens 1 T ausgesetzt wird.

2. Implantierbare medizinische Vorrichtung nach Anspruch 1, wobei:
Die Magnetvorrichtung in einer Richtung der kurzen Achse magnetisiert ist.

3. Implantierbare medizinische Vorrichtung nach Anspruch 1, wobei:
die Vorrichtung in Bezug auf eine erste Achse parallel zu einer Basis des Trägerkörpers so konfiguriert ist, dass sich die Magnetvorrichtung um die erste Achse parallel zur Basis drehen kann.

4. Implantierbare medizinische Vorrichtung nach Anspruch 3, wobei:
die Vorrichtung so konfiguriert ist, dass sich die Magnetvorrichtung um eine zweite Achse senkrecht zu einer ersten Achse und parallel zu der Basis drehen kann, oder
die Vorrichtung so konfiguriert ist, dass sie eine Drehung um eine zweite Achse senkrecht zur Basis und senkrecht zur ersten Achse wirksam verhindert.

5. Implantierbare medizinische Vorrichtung nach Anspruch 1, wobei:
der Abstand der langen Achse mindestens 33% größer ist als der Abstand der kurzen Achse.

6. Implantierbare medizinische Vorrichtung nach Anspruch 1, wobei:
die implantierbare medizinische Vorrichtung ein Empfängerstimulator eines Cochlea-Implantats ist; und
die Magnetvorrichtung einen maximalen Durchmesser von nicht mehr als 8 mm und/oder einen minimalen Durchmesser von nicht weniger als 3 mm aufweist.

7. Implantierbare medizinische Vorrichtung nach Anspruch 1, wobei:
die Magnetvorrichtung eine nicht-sphärische Magnetvorrichtung ist;
die Vorrichtung so konfiguriert ist, dass sie die Magnetvorrichtung in die Lage versetzt, sich relativ zu dem Trägerkörper zu drehen, wenn sie einem externen Magnetfeld ausgesetzt ist, so dass sich ein Magnetfeld der Magnetvorrichtung stärker auf das externe Magnetfeld ausrichtet, als dies sonst der Fall wäre, und
mindestens eines des Folgenden:
die Magnetvorrichtung ist eine modifizierte Kugelform; oder
die Magnetvorrichtung ist so konfiguriert, dass sie sich relativ zum Trägerkörper um mehr als eine Achse dreht.

8. Implantierbare medizinische Vorrichtung nach Anspruch 7, wobei:
die medizinische Vorrichtung so konfiguriert ist, dass in einer entspannten Position eine lange Achse der Magnetvorrichtung relativ parallel zu einer Oberfläche der Haut unmittelbar über der Magnetvorrichtung ist, wenn die Vorrichtung zwischen Knochen und der Oberfläche der Haut implantiert ist.

9. Implantierbare medizinische Vorrichtung nach Anspruch 8, wobei:
die medizinische Vorrichtung so konfiguriert ist, dass ein 3-T-Magnetfeld, das parallel zur Oberfläche der Haut unmittelbar über der Magnetvorrichtung ausgerichtet ist, die Längsachse der Magnetvorrichtung relativ senkrecht zur Oberfläche der Haut bewegt, wenn die Vorrichtung zwischen Knochen und der Oberfläche der Haut implantiert ist, und
die medizinische Vorrichtung so konfiguriert ist, dass nach dem Wegfall des 3-T-Magnetfelds die Magnetvorrichtung die lange Achse der Magnetvorrichtung zurück in Richtung der relativ parallelen Ausrichtung zur Hautoberfläche bewegt, wenn die Vorrichtung zwischen Knochen und der Hautoberfläche implantiert ist.

10. Implantierbare medizinische Vorrichtung nach Anspruch 7, wobei:
der Körper elastische Merkmale aufweist, die die Magnetvorrichtung mit einer langen Achse relativ parallel zur Oberfläche der Haut bei Abwesenheit des 3-T-Magnetfeldes halten und die lange Achse in die relativ parallele Ausrichtung zurückführen, wenn das 3-T-Magnetfeld ausgeschaltet wird.

11. Implantierbare medizinische Vorrichtung nach Anspruch 7, wobei:
die Vorrichtung so konfiguriert ist, dass die Magnetvorrichtung innerhalb des Trägerkörpers taumeln kann.

12. Implantierbare medizinische Vorrichtung nach Anspruch 8, wobei:
eine Platte im Inneren des Trägerkörpers zwischen der Magnetvorrichtung und der Hautoberfläche angeordnet ist, wobei die Platte bei einer Drehung der Magnetvorrichtung die Kraft im gesamten Körper im Vergleich zu dem, was sonst der Fall wäre, verteilt, und/oder
die Magnetvorrichtung einen kreisförmigen Querschnitt aufweist, der auf einer ersten Ebene senkrecht zu einer Nord-Süd-Magnetisierungsrichtung der Magnetvorrichtung liegt, und einen nicht kreisförmigen und nicht flachen Querschnitt aufweist, der auf einer zweiten Ebene senkrecht zu der ersten Ebene liegt.

13. Implantierbare medizinische Vorrichtung nach einem der Ansprüche 1-12, wobei:
die Vorrichtung so konfiguriert ist, dass eine obere Umkehrpunt Position der Magnetvorrichtung vermieden wird.

## Revendications

1. Dispositif médical implantable (100), comprenant :
un appareil à aimant (1600) ; et
un corps de support supportant l'appareil à aimant,
l'appareil à aimant ayant un axe long et un axe court plus court que l'axe long perpendiculaire à l'axe long, et
au moins l'une de la surface supérieure ou de la surface inférieure de l'appareil à aimant établissant une périphérie externe incurvée par rapport à une section transversale reposant sur un plan sur lequel repose l'axe long et qui est parallèle à l'axe court,
**caractérisé en ce que**
le corps de support inclut une portion monolithique constituée d'un matériau élastomère (199) qui enveloppe au moins partiellement l'appareil à aimant et qui positionne l'appareil à aimant de sorte que l'appareil à aimant est incliné dans un sens tel que l'axe long est généralement parallèle à une base du dispositif, ou
le corps de support inclut une portion monolithique constituée de matériau élastomère (199) qui enveloppe au moins partiellement l'appareil à aimant et se déforme élastiquement pour permettre à l'appareil à aimant de tourner dans un plan perpendiculaire à une base du dispositif au moins à 45, 60, 70, 85 ou 90 degrés depuis une orientation relâchée lorsque soumis à un champ magnétique d'au moins 1 T.

2. Dispositif médical implantable selon la revendication 1 :
l'appareil à aimant étant aimanté dans un sens de l'axe court.

3. Dispositif médical implantable selon la revendication 1 :
par rapport à un premier axe parallèle à une base du corps de support, le dispositif étant configuré de sorte que l'appareil à aimant peut tourner autour du premier axe parallèle à la base.

4. Dispositif médical implantable selon la revendication 3 :
le dispositif étant configuré de sorte que l'appareil à aimant peut tourner autour d'un second axe perpendiculaire à un premier axe et parallèle à la base, ou
le dispositif étant configuré pour empêcher efficacement une rotation autour d'un second axe perpendiculaire à la base et perpendiculaire au premier axe.

5. Dispositif médical implantable selon la revendication 1 :
la distance de l'axe long étant au moins 33 % plus grande que la distance de l'axe court.

6. Dispositif médical implantable selon la revendication 1 :
le dispositif médical implantable étant un récepteur-stimulateur d'un implant cochléaire ; et l'appareil à aimant ayant un diamètre maximal de pas plus de 8 mm et/ou un diamètre minimal de pas moins de 3 mm.

7. Dispositif médical implantable selon la revendication 1 :
l'appareil à aimant étant un appareil non sphérique à aimant ;
le dispositif étant configuré pour permettre à l'appareil à aimant de tourner par rapport au corps de support lorsqu'exposé à un champ magnétique externe de sorte qu'un champ magnétique de l'appareil à aimant s'aligne plus sur le champ magnétique externe par rapport à ce qui serait sinon le cas, et
au moins l'un de :
l'appareil à aimant étant d'une forme de sphère modifiée ; ou
l'appareil à aimant étant configuré pour tourner par rapport au corps de support autour de plus d'un axe.

8. Dispositif médical implantable selon la revendication 7 :
le dispositif médical étant configuré de sorte que dans une position relâchée, un axe long de l'appareil à aimant est relativement parallèle à une surface de la peau immédiatement au-dessus de l'appareil à aimant lorsque le dispositif est implanté entre l'os et la surface de la peau.

9. Dispositif médical implantable selon la revendication 8 :
le dispositif médical étant configuré de sorte qu'un champ magnétique 3 T aligné parallèle à la surface de la peau immédiatement au-dessus de l'appareil à aimant déplace l'axe long de l'appareil à aimant relativement perpendiculaire à la surface de la peau lorsque le dispositif est implanté entre l'os et la surface de la peau, et
le dispositif médical étant configuré de sorte que lors de l'élimination du champ magnétique 3 T, l'appareil à aimant déplace l'axe long de l'appareil à aimant en retour relativement parallèle à la surface d'orientation de la peau lorsque le dispositif est implanté entre l'os et la surface de la peau.

10. Dispositif médical implantable selon la revendication 7 :
le corps incluant des éléments élastiques qui maintiennent l'appareil à aimant avec un axe long relativement parallèle à la surface de la peau en l'absence du champ magnétique 3 T et ramène l'axe long vers l'orientation relativement parallèle lors de l'élimination du champ magnétique 3 T.

11. Dispositif médical implantable, selon la revendication 7 :
le dispositif étant configuré pour permettre à l'appareil à aimant de culbuter à l'intérieur du corps de support.

12. Dispositif médical implantable selon la revendication 8 :
une plaque étant localisée à l'intérieur du corps de support entre l'appareil à aimant et la surface de la peau, laquelle plaque diffuse une force à travers le corps lors de la rotation de l'appareil à aimant par rapport à ce qui serait sinon le cas, et/ou
l'appareil à aimant ayant une section transversale circulaire reposant sur un premier plan orthogonal à un sens d'aimantation nord-sud de l'appareil à aimant et une section transversale non circulaire et non plate reposant sur un second plan orthogonal au premier plan.

13. Dispositif médical implantable, selon l'une quelconque des revendications 1 à 12 :
le dispositif étant configuré pour éviter une position point mort haut de l'appareil à aimant.
